(19) Europäisches Patentamt
European Patent Office
Office européen des brevets

(11) **EP 4 234 014 A1**

(12) **EUROPEAN PATENT APPLICATION**

(43) Date of publication:
**30.08.2023 Bulletin 2023/35**

(21) Application number: **22159261.1**

(22) Date of filing: **28.02.2022**

(51) International Patent Classification (IPC):
***A61P 3/08*** (2006.01) ***A61P 9/00*** (2006.01)
***A61P 13/00*** (2006.01) ***A61P 17/06*** (2006.01)
***A61P 25/00*** (2006.01) ***A61P 27/02*** (2006.01)
***A61P 27/16*** (2006.01) ***A61P 35/00*** (2006.01)
***A61P 37/00*** (2006.01) ***C07C 271/22*** (2006.01)
***C07C 275/24*** (2006.01) ***C07D 295/205*** (2006.01)
***A61K 31/17*** (2006.01) ***A61K 31/27*** (2006.01)

(52) Cooperative Patent Classification (CPC):
**C07D 295/205; A61P 3/08; A61P 9/00; A61P 13/00; A61P 17/06; A61P 25/00; A61P 27/02; A61P 27/16; A61P 35/00; A61P 37/00; C07C 271/22; C07C 275/24**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Insusense ApS**
**2100 København (DK)**

(72) Inventors:
- **LITTLE, Paul Brian**
  **2100 Copenhagen (DK)**
- **CASES-THOMAS, Manuel Javier**
  **2100 Copenhagen (DK)**
- **KJØLBY, Mads Fuglsang**
  **2100 Copenhagen (DK)**
- **NYKJÆR, Anders**
  **2100 Copenhagen (DK)**

(74) Representative: **Gill Jennings & Every LLP**
**The Broadgate Tower**
**20 Primrose Street**
**London EC2A 2ES (GB)**

Remarks:
The complete document including Reference Table(s) and the Sequence Listing(s) can be downloaded from the EPO website

# (54) AMINO ACID BASED CARBAMATES AND/OR UREAS FOR THE TREATMENT OF SORTILIN DEPENDENT DISEASES

(57) The present invention relates to compounds of formula (I), which are modulators of sortilin activity, pharmaceutical compositions comprising these compounds and the use of these compounds in the treatment or prevention of medical conditions where modulation of sortilin activity is beneficial.

EP 4 234 014 A1

## Description

### FIELD OF THE INVENTION

The invention relates to compounds that bind to and modulate the activity of sortilin and pharmaceutical compositions comprising these compounds. The invention also relates to their use in treating or preventing medical conditions where modulation of sortilin activity is beneficial.

### BACKGROUND

Sortilin is a Type I transmembrane protein that acts as a receptor of several ligands (Petersen *et al.,* 1997). Sortilin is abundantly expressed in neurons and microglia of the nervous system, the inner ear, and in some peripheral tissues involved in metabolic control (Tauris *et al.,* 2020; Goettsch *et al.,* 2017; Willnow *et al.,* 2011; Kjolby *et al.,* 2010). Besides acting as a receptor involved in signalling, sortilin mediates sorting of select cargo between the cell surface trans-Golgi network, and endosomal pathway (Nykjaer & Willnow, 2012; Willnow, Petersen, & Nykjaer, 2008). Sortilin harbours a large extracellular domain denoted VPS10 that defines the family of receptors named sortilins or VS10p domain receptors. The VPS10P domain in sortilin is homologous to yeast VPS10P and is made up by a 10-bladed beta-propeller structure and a cysteine-rich 10CC module (Nykjaer & Willnow, 2012; Zheng, Brady, Meng, Mao, & Hu, 2011).

Sortilin binds multiple ligands, including pro-nerve growth factor (pro-NGF), pro-BDNF, pro-neurotrophin-3, neurotensin, and ApoB (Chen et al., 2005; Kjolby et *al.,* 2010; Mazella *et al.,* 1998; Nykjaer *et al.,* 2004; Quistgaard *et al.,* 2009; Yano, Torkin, Martin, Chao, & Teng, 2009). Furthermore, Sortilin binds progranulin (PGRN), a secreted protein involved in many cellular functions including securing lysosomal processes, anti-inflammatory responses, and neurotrophic stimulation (Galimberti, Fenoglio, & Scarpini, 2018). Sortilin targets PGRN for rapid endocytosis and degradation, and it is now well established that sortilin is the most important clearance receptor for PGRN (Hu *et al.,* 2010). Thus, sortilin negatively regulates the extracellular levels of PGRN in the periphery as well as in the brain. Indeed, lack of or blocking the receptor increases plasma PGRN levels both in mice and humans (Carrasquillo et al., 2010; Gass, Prudencio, Stetler, & Petrucelli, 2012; Hu *et al.,* 2010; Lee *et al.,* 2014; Miyakawa *et al.,* 2020; Pottier *et al.,* 2018).

Frontotemporal dementia is a highly heritable dementia and haploinsufficiency of the PGRN gene accounts for up to 25% of all cases (Gijselinck, Van Broeckhoven, & Cruts, 2008). Patients with heterozygous loss-of-function mutations in PGRN have >50% reduced extracellular levels of the protein and will invariably develop FTD, making PGRN a causal gene for the disease (Baker *et al.,* 2006; Carecchio *et al.,* 2011; Cruts & Van Broeckhoven, 2008; Galimberti *et al.,* 2010). In addition, PGRN mutant alleles have been identified in Alzheimer's (AD) patients (Brouwers *et al.,* 2008; Sheng, Su, Xu, & Chen, 2014) and high levels of extracellular PGRN are protective in models of ALS, Parkinson's disease, stroke, arthritis, and atherosclerosis (Egashira *et al.,* 2013; Laird *et al.,* 2010; Martens *et al.,* 2012; Tang *et al.,* 2011; Tao, Ji, Wang, Liu, & Zhu, 2012; Van Kampen, Baranowski, & Kay, 2014).

Sortilin is, however, not required for PGRN to elicit its functions. Hence, neurons devoid in sortilin expression are equally responsive to PGRN-induced neuronal outgrowth (De Muynck et al., 2013; Gass, Lee, et al., 2012). Further, PGRN is successfully delivered to neuronal lysosomes in sortilin-deficient cells, suggesting the existence of alternative trafficking pathways. Indeed, PGRN can bind to the lysosomal protein, prosaposin (PSAP). When PSAP binds to its cognate receptors, the cation-independent mannose-6-phosphate receptor and LRP1, it brings along PGRN to the lysosomes (Zhou et al., 2015). Finally, in a phase II clinical trial with a monoclonal anti-sortilin antibody, markers for lysosomal integrity were normal (NCT03987295).

The functional PGRN receptor remains to be identified. However, studies suggest that PGRN promotes neuronal survival, reduces inflammation and increases Aβ endocytosis by microglia (Martens et al., 2012; Pickford et al., 2011; Yin et al., 2010).

Binding of PGRN to sortilin requires the three amino acids in the C-terminal of PGRN (QLL in human, PLL in mouse), and a peptide derived from the last 24 amino acids of PGRN binds with similar affinity as the full-length protein (Zheng *et al.,* 2011). It was proposed that this mode of binding is structurally similar to Neurotensin binding (Zheng *et al.,* 2011), i.e. binding in the NTIS1 binding site of sortilin. There has been a successful small molecule screen that identified a blocker of Neurotensin to sortilin binding done in collaboration with Aarhus University (Andersen *et al.,* 2014; Schroder *et al.,* 2014).

Sortilin exists as a full-length and sorting competent receptor but is also capable of forming multimeric signalling receptor-ligand. Portions of sortilin can also be liberated from the plasma membrane to scavenge ligands (NT in pain) and control the activity of ligands. For example, sortilin is involved in synaptic plasticity by controlling the conversion rate of pro-BDNF into BDNF. This may also apply to other proneurotrophins.

Finally, the propeptide of sortilin, also named spadin, that is a ligand of the receptor has been demonstrated to control the activity of the membrane transporter TREK-1, which is a target for major depression, among other diseases. Structurally, sortilin has an amino acid sequence according to SEQ ID NO: 1 and comprises a signal peptide, a propeptide,

the Vps10p domain, a 10cc domain (10CCa + 10CCb), a transmembrane domain and a cytoplasmic tail. The luminal domain of sortilin has 6 potential *N*-linked glycosylation sites, whilst the cytoplasmic tail enables for the recruitment of various adapter proteins.

Sortilin binds to a vast number of ligands and membrane receptors and as a result engages in functions known to be important in cellular signalling and sorting. For example, sortilin is involved in signalling by proneurotrophins: the proforms of nerve growth factor (pro-NGF), brain derived neurotrophic factor (pro-BDNF), and neurotrophin-3 (proNT3), respectively. In complex with the protein p75NTR (p75 neurotrophin receptor), sortilin has been reported to form the receptor for proneurotrophin-mediated apoptotic effects leading to degeneration and cell death in cellular and animal models (Jansen *et al.,* 2007; Tenk *et al.,* 2005; Nykjaer *et al.,* 2004).

Previous work has suggested a role for sortilin in cellular sorting and signalling associated with diseases such as diabetes and obesity (Huang *et al.,* 2013). Sortilin facilitates translocation of GLUT4 to the plasma membrane and rescues it from degradation in the lysosomes (Pan *et al.,* 2017). Sortilin levels have been shown to be modulated by the level of inflammation associated with these diseases. The pro-inflammatory cytokine, TNF$\alpha$, reduces both mRNA levels and protein levels of sortilin in cultured mouse and human adipocytes, as well as *in vivo* when injected into mice (Kaddai *et al.,* 2009). Sortilin can also influence cytokine secretion: targeting sortilin in immune cells has been proposed to attenuate inflammation and reduce atherosclerosis disease progression (Mortensen *et al.,* 2014). Additionally, US 2016/0331746 describes various scaffolds of small molecules capable of binding to the active site of sortilin. Sortilin is involved in the regulation of glucose uptake (Shi & Kandror. 2005) and the development of lipid disorder diseases (Gao *et al.,* 2017).

Further, plasma sortilin levels have been reported to be a potential biomarker for identifying patients with either coronary heart disease or diabetes mellitus (Oh *et al*., 2017; Møller *et al.,* 2021). Patients that showed increased sortilin levels within their plasma, and therefore identifiable as suffering from the above conditions, also displayed enhanced glucose levels suggesting sortilin as a therapeutic target for treating these conditions. Soluble sortilin is also proposed as a treatment for type II diabetes (WO2021116290 A1, 2021).

Sortilin has been linked to various conditions affecting the central nervous system (CNS). Some studies suggest a role of circulating sortilin in patients with psychiatric disorders such as depression, which may relate to altered activity of neurotrophic factors (Buttenshon *et al.,* 2015); and it has also been reported that sortilin plays a role in brain aging, Alzheimer's Disease and frontotemporal dementia (Xu *et al.,* 2019). However, delivering therapeutic agents that are capable of crossing the blood-brain barrier to the CNS presents a major challenge.

The blood-brain barrier is a highly selective semipermeable border of endothelial cells that prevents solutes in the circulating blood from non-selectively crossing into the extracellular fluid of the CNS where neurons reside. Therapy of neurological diseases is therefore limited due to the restricted penetration of therapeutic agents across the blood-brain barrier.

Therapeutic agents for treating CNS diseases therefore must be capable of crossing the blood-brain barrier. In addition to this, they must also have a sufficient unbound drug concentration in the brain, as the free drug hypothesis states that only unbound compound is able to interact with and elicit a pharmacological effect.

To determine the unbound fraction of a test compound ($F_{ub}$), sample supernatants may be analysed by methods such as liquid chromatography with tandem mass spectrometry (LC-MS/MS). The unbound fraction may then be calculated from the peak area ratios obtained for each matrix according to the following formula:

$$F_{ub} = C_{PBS} / C_{plasma}$$

where $C_{PBS}$ and $C_{plasma}$ are the analyte concentrations in PBS (receiver) and plasma (donor), respectively.

Recovery samples may be prepared in each condition but without dialysis, and may be used for evaluation of recovery from dialysis experiments using the following formula:

$$\% \text{ Recovery} = 100\times(V_{PBS}\times C_{PBS}+V_{plasma}\times C_{plasma})/V_{plasma}\times C_{recovery}$$

where $V_{PBS}$ is the volume on the receiver side (PBS) and $V_{plasma}$ is the volume on donor side (plasma) of the dialysis device. $C_{recovery}$ is the analyte concentration measured from the recovery sample. Compounds such as propranolol or fluoxetine, may be included in experiments as controls.

The unbound fraction in brain ($F_{ub,\ brain}$) may be calculated from the measured value in brain homogenate ($F_{ub,\ meas}$), taking into account the dilution factor used in preparing the brain homogenate:

$$F_{ub,\,brain} = \frac{1/D}{\left(\frac{1}{F_{ub,\,meas}}\right) - 1 + \left(\frac{1}{D}\right)}$$

where D = dilution factor.

The brain/plasma unbound partition coefficient ($K_{puu}$) may be determined as a ratio between the free compound concentrations in plasma and brain:

$$K_{puu} = \frac{C_{ub,brain}}{C_{ub,plasma}}$$

Where $C_{u,brain}$ = unbound concentration in brain ($C \times F_{ub}$, brain); wherein

C = concentration at steady state; and

$C_{ub,plasma}$ = unbound concentration in plasma ($C \times F_{ub}$).

For the treatment of CNS diseases, it is desirable for the $K_{puu}$ to have the highest possible value, above 0. A value around 1 indicating that the free fraction compound freely permeates the blood brain barrier; a value above 1 suggesting that an active influx transport mechanism at the blood brain barrier is involved; and less than 1, which indicates that the free fraction compound is either poorly permeable or is recognized by an active efflux mechanism, reducing the exposure in the CNS while passing back through the blood-brain barrier to plasma or the CSF. A $K_{puu}$ value of 0 or close to 0, indicates a poorly permeable compound or a highly active efflux mechanism that in any case will make highly improbable to reach a meaningful exposure in the CNS of the desired active species.

In view of the above, there is an unmet need for new compounds that may be used in the treatment and prevention of medical conditions in which modulation of sortilin is beneficial, including a neurodegenerative disorder, a psychiatric disorder, an inflammatory disorder, a cancer, pain, diabetes mellitus, brain tumours, diabetic retinopathy, glaucoma, chronic pain, uveitis, cardiovascular disease, kidney disease, psoriasis, hereditary eye conditions, hearing loss or diseases characterized by misfolded tau. The neurodegenerative disorder may be selected from frontotemporal dementia, Alzheimer's disease, Parkinson's disease, Huntington's disease, prion diseases such as Creutzfeldt-Jakob disease (CJD), acute brain injury, spinal cord injury and stroke. The psychiatric disorder may be selected from bipolar disorder, major depression, post-traumatic stress disorder, and anxiety disorders. The inflammatory disorder may be selected from inflammatory diseases and neuroinflammation. The cancer may be selected from breast cancer, lung cancer, ovarian cancer, prostate cancer, thyroid cancer, pancreatic cancer, glioblastoma and colorectal cancer. The hearing loss may be selected from noise-induced hearing loss, ototoxicity induced hearing loss, age-induced hearing loss, idiopathic hearing loss, tinnitus and sudden hearing loss.

There is also an unmet need for sortilin modulators that are capable of crossing the blood brain barrier and that have a $K_{puu}$ greater than 0.1 for use in the treatment of a disease of the central nervous system.

## SUMMARY OF THE INVENTION

The invention relates to compounds of formula (I), pharmaceutical compositions comprising these compounds and the use of these compounds and pharmaceutical compositions for use in therapy. The therapy may involve the treatment or prevention of medical conditions where modulation of sortilin activity is beneficial. Such medical conditions include a neurodegenerative disorder, a psychiatric disorder, an inflammatory disorder, a cancer, pain, diabetes mellitus, brain tumours, diabetic retinopathy, glaucoma, chronic pain, uveitis, cardiovascular disease, kidney disease, psoriasis, hereditary eye conditions, hearing loss, diseases characterized by misfolded tau or a disease of the central nervous system.

The compounds according to the invention may have a blood-to-brain $K_{puu}$ of more than 0.1, and are therefore capable of crossing the blood-brain-barrier. Compounds exhibiting this property may be used in the treatment or prevention of a disease of the central nervous system.

## DETAILED DESCRIPTION

In a first aspect, the present invention provides a compound of Formula (I)

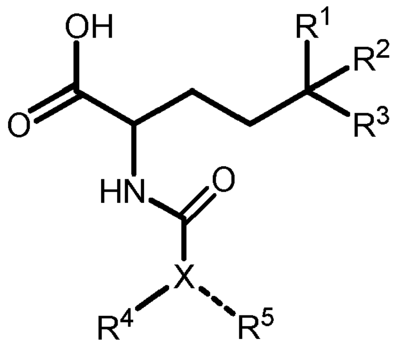

(I)

or a pharmaceutically acceptable salt, solvate, hydrate, tautomer, optical isomer, N-oxide, and/or prodrug thereof; wherein

X is selected from N and O;

$R^1$, $R^2$ and $R^3$ are each independently selected from the group consisting of halo, H, $(C_1-C_4)$alkyl, halo-$(C_1-C_4)$alkyl, $(C_2-C_4)$alkenyl, and halo-$(C_2-C_4)$alkenyl;

and

wherein $R^4$ is selected from the group consisting of $(C_3-C_{20})$aryl, halo-$(C_3-C_{20})$aryl, $(C_3-C_8)$heteroaryl, halo-$(C_3-C_{20})$heteroaryl, $(C_1-C_6)$-alkylene-$(C_3-C_{20})$-aryl, $(C_1-C_6)$-alkylene-$(C_3-C_{20})$-heteroaryl, $(C_1-C_6)$-alkylene-(3- to 10- membered-heterocyclic ring);

wherein the aryl group in $(C_1-C_6)$-alkylene-$(C_3-C_{20})$-aryl, the heteroaryl group in $(C_1-C_6)$-alkylene-$(C_3-C_{20})$-heteroaryl or the heterocyclic ring in $(C_1-C_6)$-alkylene-(3- to 8- membered heterocyclic ring) is optionally substituted with one or more substituents independently selected from halo, H, -OH, $(C_1-C_4)$alkyl, halo-(Ci-$C_4$)alkyl, $(C_1-C_4)$alkoxy and halo-$(C_1-C_4)$alkoxy;

wherein when X is O, $R^5$ is not present; and

when X is N, $R^5$ is selected from selected from the group consisting of halo, H, $(C_1-C_4)$alkyl, halo-$(C_1-C_4)$alkyl, $(C_2-C_4)$alkenyl, and halo-$(C_2-C_4)$alkenyl;

or

wherein when X is N; and X, $R^4$ and $R^5$ taken together form a 4- to 20- membered heterocyclic ring; wherein the heterocyclic ring is monocyclic, bicyclic or tricyclic and is optionally substituted with one or more substituents independently selected from halo, OH, cyano, carbonyl, $(C_1-C_4)$alkyl, halo-$(C_1-C_4)$alkyl, acetyl, $(C_1-C_4)$alkoxy, and halo-$(C_1-C_4)$alkoxy.

It has been found that compounds of formula (I) are sortilin modulators which bind and modulate sortilin and therefore may be useful in conditions where sortilin inhibition is beneficial. Such conditions include a neurodegenerative disorder, a psychiatric disorder, an inflammatory disorder, a cancer, pain, diabetes mellitus, brain tumours, diabetic retinopathy, glaucoma, chronic pain, uveitis, cardiovascular disease, kidney disease, psoriasis, hereditary eye conditions, hearing loss, diseases characterized by misfolded tau or a disease of the central nervous system. The neurodegenerative disorder may be selected from frontotemporal dementia, Alzheimer's disease, Parkinson's disease, Huntington's disease, prion diseases such as Creutzfeldt-Jakob disease (CJD), acute brain injury, spinal cord injury and stroke; the psychiatric disorder may be selected from bipolar disorder, major depression, post-traumatic stress disorder, and anxiety disorders; the inflammatory disorder may be selected from inflammatory diseases and neuroinflammation; the cancer may be selected from breast cancer, lung cancer, ovarian cancer, prostate cancer, thyroid cancer, pancreatic cancer, glioblastoma and colorectal cancer; and the hearing loss may be selected from noise-induced hearing loss, ototoxicity induced hearing loss, age-induced hearing loss, idiopathic hearing loss, tinnitus and sudden hearing loss.

As used herein, the term "sortilin" may refer to full length sortilin (also referred to as immature sortilin), comprising

a signal peptide, a propeptide, a Vps10p domain, a 10CC domain, a transmembrane domain and a large cytoplasmic tail. The term "sortilin" may have an amino acid sequence according to SEQ ID NO: 1 or SEQ ID NO: 2 as defined in European Patent Application No. 21152638.9. Alternatively, it may refer to mature sortilin, comprising a Vps10p domain, a 10CC domain, a transmembrane domain and a large cytoplasmic tail, having an amino acid sequence according to SEQ ID NO: 3 as defined in European Patent Application No. 21152638.9, or a naturally occurring fragment, homologue or variant thereof. The term "sortilin" or "sortilin molecule" are used interchangeably herein. It is understood that sortilin is capable of interacting with a pro-neurotrophin molecule to form a sortilin/pro-neurotrophin complex. This sortilin/pro-neurotrophin complex may or may not be capable of interacting with a p75NTR molecule to form a trimeric complex comprising sortilin, pro-neurotrophin and p75NTR. It is understood that this trimeric complex may be responsible for adverse biological responses, such as stimulating apoptosis in retinal and ganglion cells, and controlling growth cone retraction of projecting axons (Jansen *et al.,* 2007; Nykjaer *et al.,* 2004; Santos et al., 2012; Skeldal *et al.,* 2012).

As used herein, the term "pro-neurotrophin" refers to the larger precursors of neurotrophins, which undergo proteolytic cleavage to yield the mature form of the neurotrophin. Neurotrophins are a family of proteins that induce the survival, development and function of neurons, and are commonly referred to as growth factors. Pro-neurotrophins are biologically active and have distinct roles compared to their neurotrophin counterparts, such as induction of apoptosis. Examples of pro-neurotrophins include pro-NGF, pro-BDNF, proNT3 and proNT4. Proneurotrophins may also control synaptic plasticity. Whereas mature neurotrophins induce synaptic strength, in their proforms they may weaken synapses.

The compounds of the invention may be sortilin inhibitors, binders, modulators or antagonists. As used herein, the term "sortilin antagonist", "sortilin inhibitor", "sortilin binder" or "sortilin modulator" (used interchangeably) refers to a substance that interferes with, blocks, or otherwise attenuates the effect of, a sortilin protein binding to progranulin, or neurotensin or another extracellular ligand, or a proneurotrophin (e.g., pro-NGF, proNT3, pro-BDNF) or preventing the formation of the trimeric complex between sortilin, p75NTR and the pro-neurotrophin. The term "sortilin antagonist" also includes a substance or agent that interferes with the formation of a high affinity trimeric complex. In the latter scenario, it is recognised that a trimeric complex may be formed in that sortilin can bind to p75NTR (but not pro-NGF) and p75NTR can simultaneously bind the NGF domain of pro-NGF. However, the resulting trimeric complex may be of lower affinity for its receptor and as a result have significantly reduced capacity to stimulate apoptosis via the mechanism described above. Skeldal *et al.* (2012) demonstrated that the apoptotic function of the trimeric complex is abolished when sortilin is devoid in its intracellular domain. The term "sortilin antagonist" also includes a substance or agent that interferes with, blocks, or otherwise attenuates the effect of, a sortilin protein interacting with p75NTR. This interaction may be completely prevented, in which case the trimeric complex is prevented from forming, or only partially prevented, in which case the trimeric complex may be formed but may have reduced biological potency. Skeldal *et al* showed that complex formation between sortilin and p75NTR relies on contact points in the extracellular domains of the receptors and that the interaction critically depends on an extracellular juxtamembrane 23-amino acid sequence of p75NTR. Thus, the sortilin antagonist may interfere with this 23-amino acid sequence or proximal sequences in the molecules. "Sortilin antagonists" may act as ligand cellular uptake inhibitors wherein the ligands may be progranulin, neurotensin or BDNF.

It is preferred that $R^1$, $R^2$ and $R^3$ are each independently selected from the group consisting of halo, $(C_1\text{-}C_2)$alkyl and halo-$(C_1\text{-}C_2)$alkyl.

The alkyl and haloalkyl groups may be linear or branched.

In a preferred aspect of the invention, $R^1$, $R^2$ and $R^3$ are each independently selected from F, $CH_3$ and $CF_3$. Most preferably, $R^1$, $R^2$ and $R^3$ are the same. For example, in an exemplary compound of the invention, $R^1$, $R^2$ and $R^3$ may each be F, $CH_3$ or $CF_3$.

In another preferred aspect of the invention, $R^4$ is selected from the group consisting of $(C_4\text{-}C_{10})$aryl, halo-$(C_4\text{-}C_{10})$aryl, $(C_3\text{-}C_8)$heteroaryl, halo-$(C_3\text{-}C_{20})$heteroaryl, $(C_1\text{-}C_4)$-alkylene-$(C_4\text{-}C_{10})$-aryl, $(C_1\text{-}C_4)$-alkylene-$(C_4\text{-}C_{10})$-heteroaryl, and $(C_1\text{-}C_4)$-alkylene-(3- to 10- membered-heterocyclic ring).

The heteroaryl may comprise one, two or more heteroatoms. Preferably, the heteroaryl comprises one or two heteroatoms. The heteroatom may be selected from N, S or O. In groups with more than one heteroatom present, the heteroatoms may be the same or they may be different.

The aryl and heteroaryl groups may be monocyclic or bicyclic, preferably monocyclic. Preferably, the aryl and heteroaryl groups have between 5-8 carbon atoms. The heteroaryl group may have a ring size of 6-12 members, preferably 6-8 members.

The alkylene parts of the alkylene-aryl, alkylene-heteroaryl, and alkylene-(3- to 10- membered-heterocyclic ring) may be linear or branched. They may be optionally substituted with halogen groups such as F.

In some preferred embodiments, $R^4$ is

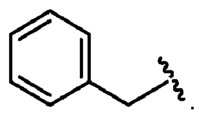

In another preferred aspect of the invention, X is N and $R^5$ is selected from the group consisting of H, ($C_1$-$C_3$)-alkyl and ($C_1$-$C_3$)haloalkyl.

In another preferred aspect of the invention, X is N; and X, $R^4$ and $R^5$ taken together form a 5- to 10- membered heterocyclic ring; wherein the heterocyclic ring is monocyclic or bicyclic and is optionally substituted with one or more substituents independently selected from halo, OH, carbonyl, ($C_1$-$C_3$)alkyl, halo-($C_1$-$C_3$)alkyl, acetyl and ($C_1$-$C_3$)alkoxy, and halo-($C_1$-$C_3$)alkoxy.

The alkyl, haloalkyl, alkoxy and haloalkoxy substituents may be linear or branched.

The substituent may be attached at any position of the aryl, heteroaryl or heterocyclic ring. The one or more substituents may be attached to a carbon atom, heteroatom or combinations thereof. Preferably, there are no substituents or between one to five substituents.

The heteroaryl or heterocyclic ring may comprise one, two or more heteroatoms. Preferably, the heteroaryl or heterocyclic ring comprises one or two heteroatoms. The heteroatom may be selected from N, S or O. In groups with more than one heteroatom present, the heteroatoms may be the same or they may be different.

The heterocyclic ring may be aliphatic. It may be monocyclic, bicyclic or tricyclic. Preferably, the heterocyclic ring is monocyclic or bicyclic. Preferably, the heterocyclic ring has between 5-10 members, more preferably between 5-9 members.

The aryl and heteroaryl groups may also be monocyclic, bicyclic or tricyclic. Preferably, monocyclic or bicyclic. Preferably, the aryl and heteroaryl groups have a ring size of between 5-10 members.

In some preferred examples of this embodiment, X is N; and X, $R^4$ and $R^5$ taken together form:

(i) 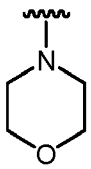 or (ii) 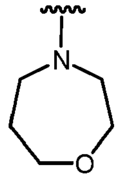 .

Particular compounds of the invention are those listed below.

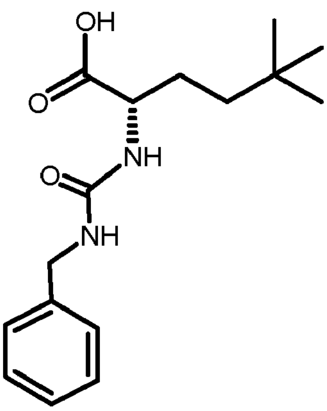

(2S)-2-[(benzylcarbamoyl)amino]-5,5-dimethylhexanoic acid;

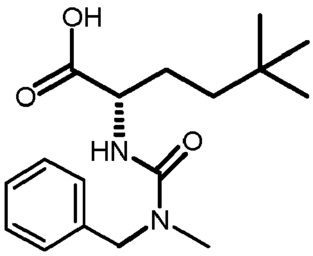

(2S)-2-{[benzyl(methyl)carbamoyl]amino}-5,5-dimethylhexanoic acid;

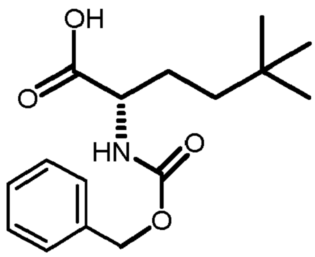

(2S)-2-{[(benzyloxy)carbonyl]amino}-5,5-dimethylhexanoic acid;

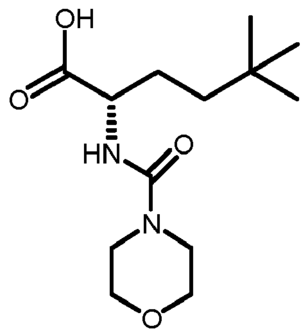

(2S)-5,5-dimethyl-2-[(morpholine-4-carbonyl)amino]hexanoic acid; or

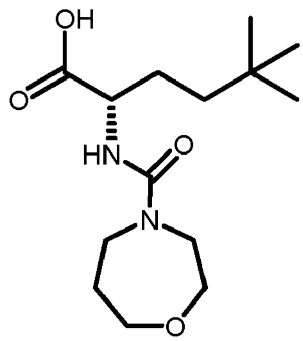

(2S)-5,5-dimethyl-2-[(1,4-oxazepane-4-carbonyl)amino]hexanoic acid.

Preferably, the compounds of formula (I) have the following configuration.

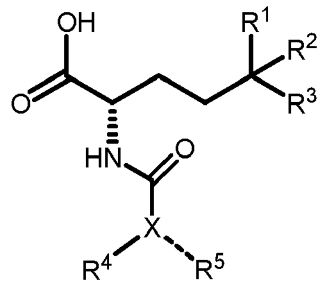

The inventors have found that the compounds may not be only effective sortilin modulators but may also be able to cross the blood brain barrier. This provides opportunities for developing more effective therapies for diseases of the central nervous system.

The compounds of the invention may have a blood-to-brain $K_{puu}$ of more than 0.1. Such compounds are capable of crossing the blood brain barrier and are suitable for use in the treatment of a disease of the central nervous system, as described herein.

The compounds may have a $K_{puu}$ of between 0.1 and 10, between 0.1 and 5, between 0.1 and 3, between 0.1 and 2, between 0.1 and 1, between 0.1 and 0.8, between 0.1 and 0.6, between 0.1 and 0.5, between 0.1 and 0.4, between 0.1 and 0.3, or between 0.1 and 0.2.

Alternatively, the compounds of the invention are not capable of crossing the blood brain barrier. Therefore, the compounds of the invention may have a $K_{puu}$ below 0.1.

Methods of measuring the blood-to-brain $K_{puu}$ are known to those skilled in the art. These include, for example, the method set out in Example 20 of European Patent Application No. 21194937.5.

According to a second aspect of the invention, there is provided a pharmaceutical composition comprising a compound according to the first aspect of the invention and one or more pharmaceutically acceptable carriers, excipients, and/or diluents.

According to a third aspect of the invention, there is provided a compound according to the first aspect of the invention or a pharmaceutical composition according to the second aspect of the invention, for use in therapy.

In a fourth aspect, there is provided a compound or pharmaceutical composition for use in the treatment or prevention of a neurodegenerative disorder, a psychiatric disorder, an inflammatory disorder, a cancer, pain, diabetes mellitus, brain tumours, diabetic retinopathy, glaucoma, chronic pain, uveitis, cardiovascular disease, kidney disease, psoriasis, hereditary eye conditions, hearing loss, diseases characterized by misfolded tau or a disease of the central nervous system.

Preferably, the neurodegenerative disorder is be selected from frontotemporal dementia, Alzheimer's disease, Parkinson's disease, Huntington's disease, prion diseases such as Creutzfeldt-Jakob disease (CJD), acute brain injury, spinal cord injury and stroke.

The psychiatric disorder is preferably selected from bipolar disorder, major depression, post-traumatic stress disorder, and anxiety disorders.

Preferably, the inflammatory disorder is selected from inflammatory diseases and neuroinflammation.

Preferably, the cancer is selected from breast cancer, lung cancer, ovarian cancer, prostate cancer, thyroid cancer, pancreatic cancer, glioblastoma and colorectal cancer.

The hearing loss is preferably selected from noise-induced hearing loss, ototoxicity induced hearing loss, age-induced hearing loss, idiopathic hearing loss, tinnitus and sudden hearing loss.

Thus, in an embodiment, the compounds for use according to the invention may disrupt interaction between a sortilin molecule and a pro-neurotrophin molecule or disrupt the interaction between a sortilin molecule and a p75NTR molecule. Said sortilin molecule may be mature sortilin.

In one embodiment according to the fourth aspect, there is provided a compound or pharmaceutical composition for use in the treatment or prevention of a disease of the central nervous system, wherein the compound has a blood-to-brain $K_{puu}$ of more than 0.1.

The compound may have a $K_{puu}$ of between 0.1 and 10, between 0.1 and 5, between 0.1 and 3, between 0.1 and 2, between 0.1 and 1, between 0.1 and 0.8, between 0.1 and 0.6, between 0.1 and 0.5, between 0.1 and 0.4, between 0.1 and 0.3, or between 0.1 and 0.2.

Preferably, the disease of the central nervous system is selected from: a neurodegenerative disorder selected from frontotemporal dementia, Alzheimer's disease, Parkinson's disease, Huntington's disease, prion diseases such as Creutzfeldt-Jakob disease (CJD), acute brain injury, spinal cord injury and stroke; a psychiatric disorder selected from bipolar disorder, major depression, post-traumatic stress disorder, and anxiety disorders; hearing loss selected from noise-induced hearing loss, ototoxicity induced hearing loss, age-induced hearing loss, idiopathic hearing loss, tinnitus and sudden hearing loss; brain tumours, retinopathies, glaucoma, neuroinflammation, chronic pain and diseases characterized by misfolded tau.

According to a fifth aspect of the invention, there is provided the use of the compound according to the first aspect of the invention for the manufacture of a medicament for the treatment or prevention of a neurodegenerative disorder, a psychiatric disorder, an inflammatory disorder, a cancer, pain, diabetes mellitus, brain tumours, diabetic retinopathy, glaucoma, chronic pain, uveitis, cardiovascular disease, kidney disease, psoriasis, hereditary eye conditions, hearing loss, diseases characterized by misfolded tau or a disease of the central nervous system.

According to a sixth aspect of the invention, there is provided a method for the treatment or prevention of a disease or condition responsive to sortilin modulation comprising administering a therapeutically effective amount of the compound according to the first aspect of the invention or the pharmaceutical composition according the second aspect of the invention.

The compounds of the invention may include isotopically labelled and/or isotopically-enriched forms of the compounds. The compounds of the invention herein may contain unnatural proportions of atomic isotopes at one or more of the atoms that constitute such compounds. Examples of isotopes that can be incorporated into the disclosed compounds include isotopes of hydrogen, carbon, nitrogen, oxygen, phosphorus, sulphur, chlorine, such as $^{2}H$, $^{3}H$, $^{11}C$, $^{13}C$, $^{14}C$, $^{13}N$, $^{15}O$, $^{17}O$, $^{32}P$, $^{35}S$, $^{18}F$, $^{36}Cl$.

The compounds of the invention may be used as such or, where appropriate, as pharmacologically acceptable

salts (acid or base addition salts) thereof. The pharmacologically acceptable addition salts mentioned below are meant to comprise the therapeutically active non-toxic acid and base addition salt forms that the compounds are able to form. Compounds that have basic properties can be converted to their pharmaceutically acceptable acid addition salts by treating the base form with an appropriate acid. Exemplary acids include inorganic acids, such as hydrogen chloride, hydrogen bromide, hydrogen iodide, sulphuric acid, phosphoric acid; and organic acids such as formic acid, acetic acid, propanoic acid, hydroxyacetic acid, lactic acid, pyruvic acid, glycolic acid, maleic acid, malonic acid, oxalic acid, benzenesulphonic acid, toluenesulphonic acid, methanesulphonic acid, trifluoroacetic acid, fumaric acid, succinic acid, malic acid, tartaric acid, citric acid, salicylic acid, p-aminosalicylic acid, pamoic acid, benzoic acid, ascorbic acid and the like. Compounds that have acidic properties can be converted to their pharmaceutically acceptable basic addition salts by treating the acid form with an appropriate base. Exemplary base addition salt forms are the sodium, potassium, calcium salts, and salts with pharmaceutically acceptable amines such as, for example, ammonia, alkylamines, benzathine, and amino acids, such as, e.g. arginine and lysine. The term addition salt as used herein also comprises solvates which the compounds and salts thereof are able to form, such as, for example, hydrates, alcoholates and the like.

Throughout the present disclosure, a given chemical formula or name shall also encompass all pharmaceutically acceptable salts, solvates, hydrates, N-oxides, and/or prodrug forms thereof. It is to be understood that the compounds of the invention include any and all hydrates and/or solvates of the compound formulas. It is appreciated that certain functional groups, such as the hydroxy, amino, and like groups form complexes and/or coordination compounds with water and/or various solvents, in the various physical forms of the compounds. Accordingly, the above formulas are to be understood to include and represent those various hydrates and/or solvates.

Compounds of the invention also include tautomeric forms. Tautomeric forms result from the swapping of a single bond with an adjacent double bond together with the concomitant migration of a proton. Tautomeric forms include prototropic tautomers which are isomeric protonation states having the same empirical formula and total charge. Example prototropic tautomers include ketone - enol pairs, amide - imidic acid pairs, lactam - lactim pairs, amide - imidic acid pairs, enamine - imine pairs, and annular forms where a proton can occupy two or more positions of a heterocyclic system, for example, 1H- and 3H-imidazole, 1H, 2Hand 4H- 1,2,4-triazole, 1H- and 2H- isoindole, and 1H- and 2H-pyrazole. Tautomeric forms can be in equilibrium or sterically locked into one form by appropriate substitution.

The compounds described herein can be asymmetric (e.g. having one or more stereogenic centres). All stereoisomers, such as enantiomers and diastereomers, are intended unless otherwise indicated. Compounds of the present invention that contain asymmetrically substituted carbon atoms can be isolated in optically active or racemic forms. Methods on how to prepare optically active forms from optically active starting materials are known in the art, such as by resolution of racemic mixtures or by stereoselective synthesis. Many geometric isomers of olefins, C=N double bonds, and the like can also be present in the compounds described herein, and all such stable isomers are contemplated in the present invention. Cis- and trans-geometric isomers of the compounds of the present invention are described and may be isolated as a mixture of isomers or as separated isomeric forms.

In the case of the compounds which contain an asymmetric carbon atom, the invention relates to the D form, the L form, and D,L mixtures and also, where more than one asymmetric carbon atom is present, to the diastereomeric forms. Those compounds of the invention which contain asymmetric carbon atoms, and which as a rule accrue as racemates, can be separated into the optically active isomers in a known manner, for example using an optically active acid. However, it is also possible to use an optically active starting substance from the outset, with a corresponding optically active or diastereomeric compound then being obtained as the end product.

The term "prodrugs" refers to compounds that may be converted under physiological conditions or by solvolysis to a biologically active compound of the invention. A prodrug may be inactive when administered to a subject in need thereof, but is converted in vivo to an active compound of the invention. Prodrugs are typically rapidly transformed in vivo to yield the parent compound of the invention, e.g. by hydrolysis in the blood. The prodrug compound usually offers advantages of solubility, tissue compatibility or delayed release in a mammalian organism (see Silverman, R. B., The Organic Chemistry of Drug Design and Drug Action, 2nd Ed., Elsevier Academic Press (2004), page 498 to 549). Prodrugs of a compound of the invention may be prepared by modifying functional groups, such as a hydroxy, amino or mercapto groups, present in a compound of the invention in such a way that the modifications are cleaved, either in routine manipulation or *in vivo,* to the parent compound of the invention. Examples of prodrugs include, but are not limited to, acetate, formate and succinate derivatives of hydroxy functional groups or phenyl carbamate derivatives of amino functional groups.

The term "treatment" as used herein may include prophylaxis of the named disorder or condition, or amelioration or elimination of the disorder once it has been established. The term "prevention" refers to prophylaxis of the named disorder or condition.

Methods delineated herein include those wherein the subject is identified as in need of a particular stated treatment. Identifying a subject in need of such treatment can be in the judgment of a subject or a health care professional and can be subjective (e.g. opinion) or objective (e.g. measurable by a test or diagnostic method).

In other aspects, the methods herein include those further comprising monitoring subject response to the

treatment administrations. Such monitoring may include periodic imaging or sampling of subject tissue, fluids, specimens, cells, proteins, chemical markers, genetic materials, etc. as markers or indicators of the treatment regimen. In other methods, the subject is pre-screened or identified as in need of such treatment by assessment for a relevant marker or indicator of suitability for such treatment.

The invention provides a method of monitoring treatment progress. The method includes the step of determining a level of diagnostic marker (Marker) (e.g. any target or cell type delineated herein modulated by a compound herein) or diagnostic measurement (e.g., screen, assay) in a subject suffering from or susceptible to a disorder or symptoms thereof delineated herein, in which the subject has been administered a therapeutic amount of a compound herein sufficient to treat the disease or symptoms thereof. The level of Marker determined in the method can be compared to known levels of Marker in either healthy normal controls or in other afflicted patients to establish the subject's disease status. In preferred embodiments, a second level of Marker in the subject is determined at a time point later than the determination of the first level, and the two levels are compared to monitor the course of disease or the efficacy of the therapy. In certain preferred embodiments, a pre-treatment level of Marker in the subject is determined prior to beginning treatment according to this invention; this pre-treatment level of Marker can then be compared to the level of Marker in the subject after the treatment commences, to determine the efficacy of the treatment.

A level of Marker or Marker activity in a subject may be determined at least once. Comparison of Marker levels, e.g., to another measurement of Marker level obtained previously or subsequently from the same patient, another patient, or a normal subject, may be useful in determining whether therapy according to the invention is having the desired effect, and thereby permitting adjustment of dosage levels as appropriate. Determination of Marker levels may be performed using any suitable sampling/expression assay method known in the art or described herein. Preferably, a tissue or fluid sample is first removed from a subject. Examples of suitable samples include blood, urine, tissue, mouth or cheek cells, and hair samples containing roots. Other suitable samples would be known to the person skilled in the art. Determination of protein levels and/or mRNA levels (e.g., Marker levels) in the sample can be performed using any suitable technique known in the art, including, but not limited to, enzyme immunoassay, ELISA, radiolabelling/assay techniques, blotting/chemiluminescence methods, real-time PCR, and the like.

For clinical use, the compounds disclosed herein are formulated into pharmaceutical compositions (or formulations) for various modes of administration. It will be appreciated that compounds of the invention may be administered together with a physiologically acceptable carrier, excipient, and/or diluent (i.e. one, two, or all three of these). The pharmaceutical compositions disclosed herein may be administered by any suitable route, preferably by oral, rectal, nasal, topical (including ophthalmic, buccal and sublingual), sublingual, transdermal, intrathecal, transmucosal or parenteral (including subcutaneous, intramuscular, intravenous and intradermal) administration. Other formulations may conveniently be presented in unit dosage form, e.g., tablets and sustained release capsules, and in liposomes, and may be prepared by any methods well known in the art of pharmacy. Pharmaceutical formulations are usually prepared by mixing the active substance, or a pharmaceutically acceptable salt thereof, with conventional pharmaceutically acceptable carriers, diluents or excipients. Examples of excipients are water, gelatin, gum arabicum, lactose, microcrystalline cellulose, starch, sodium starch glycolate, calcium hydrogen phosphate, magnesium stearate, talcum, colloidal silicon dioxide, and the like. Such formulations may also contain other pharmacologically active agents, and conventional additives, such as stabilizers, wetting agents, emulsifiers, flavouring agents, buffers, and the like. Usually, the amount of active compounds is between 0.1-95% by weight of the preparation, preferably between 0.2-20% by weight in preparations for parenteral use and more preferably between 1-50% by weight in preparations for oral administration. The formulations can be further prepared by known methods such as granulation, compression, microencapsulation, spray coating, Etc. The formulations may be prepared by conventional methods in the dosage form of tablets, capsules, granules, powders, syrups, suspensions, suppositories or injections. Liquid formulations may be prepared by dissolving or suspending the active substance in water or other suitable vehicles. Tablets and granules may be coated in a conventional manner. To maintain therapeutically effective plasma concentrations for extended periods of time, compounds disclosed herein may be incorporated into slow-release formulations.

The dose level and frequency of dosage of the specific compound will vary depending on a variety of factors including the potency of the specific compound employed, the metabolic stability and length of action of that compound, the patient's age, body weight, general health, sex, diet, mode and time of administration, rate of excretion, drug combination, the severity of the condition to be treated, and the patient undergoing therapy. The daily dosage may, for example, range from about 0.001 mg to about 100 mg per kilo of body weight, administered singly or multiply in doses, e.g. from about 0.01 mg to about 25 mg each. Normally, such a dosage is given orally but parenteral administration may also be chosen.

## DEFINITIONS

"Optional" or "optionally" means that the subsequently described event or circumstance may, but need not, occur, and that the description includes instances where the event or circumstance occurs and instances in which it

does not.

The term "heteroatom" means O, N, or S.

The term "($C_1$-$C_n$)alkyl" denotes a straight, branched or cyclic or partially cyclic alkyl group having from 1 to n carbon atoms, i.e. 1, 2, 3 or n carbon atoms. For the "($C_1$-$C_n$)alkyl" group to comprise a cyclic portion it should be formed of at least three carbon atoms. For parts of the range "($C_1$-$C_n$)alkyl" all subgroups thereof are contemplated. For example, in the range ($C_1$-$C_6$)alkyl, all subgroups such as ($C_1$-$C_5$)alkyl, ($C_1$-$C_4$)alkyl, ($C_1$-$C_3$)alkyl, ($C_1$-$C_2$)alkyl, ($C_1$)alkyl, ($C_2$-$C_6$)alkyl, ($C_2$-$C_5$)alkyl, ($C_2$-$C_4$)alkyl, ($C_2$-$C_3$)alkyl, ($C_2$)alkyl, ($C_3$-$C_6$)alkyl, ($C_3$-$C_5$)alkyl, ($C_3$-$C_4$)alkyl, ($C_3$)alkyl, ($C_4$-$C_6$)alkyl, ($C_4$-$C_5$)alkyl, ($C_4$)alkyl, ($C_5$-$C_6$)alkyl, ($C_6$)alkyl. Examples of "$C_1$-$C_6$ alkyl" include methyl, ethyl, n-propyl, isopropyl, cyclopropyl, n-butyl, isobutyl, sec-butyl, t-butyl, cyclobutyl, cyclopropylmethyl, branched or cyclic or partially cyclic pentyl and hexyl Etc.

The term "halo-($C_1$-$C_n$)alkyl" denotes a $C_1$-$C_n$ alkyl as described above substituted with at least one halogen atom, which is preferably, F, Cl, Br and I, more preferably F and Cl, and most preferably F.

When a term denotes a range, for instance "1 to 6 carbon atoms" in the definition of ($C_1$-$C_6$)alkyl, each integer is considered to be disclosed, i.e. 1, 2, 3, 4, 5 and 6.

The term "($C_2$-$C_n$)alkenyl" denotes a straight, branched or cyclic or partially cyclic alkyl group having at least one carbon-carbon double bond, and having from 2 to 6 carbon atoms. The alkenyl group may comprise a ring formed of 3 to 6 carbon atoms. For parts of the range "($C_2$-$C_n$)alkenyl" all subgroups thereof are contemplated. For example, the range "($C_2$-$C_4$)alkenyl" covers ($C_2$-$C_4$)alkenyl, ($C_2$-$C_3$)alkenyl, ($C_2$)alkenyl. Examples of "($C_2$-$C_4$)alkenyl" include 2-propenyl, 2-butenyl, 3-butenyl or 2-methyl-2-propenyl.

The term "($C_1$-$C_4$)alkoxy" denotes -O-(($C_1$-$C_4$)alkyl) in which a ($C_1$-$C_4$)alkyl group is as defined above and is attached to the remainder of the compound through an oxygen atom. Examples of "($C_1$-$C_4$)alkoxy" include methoxy, ethoxy, n-propoxy, isopropoxy, n-butoxy, isobutoxy, sec-butoxy and *t*-butoxy.

The term "halo($C_1$-$C_4$)alkoxy" denotes a ($C_1$-$C_4$)alkoxy as described above substituted with a halogen atom, which is preferably, F, Cl, Br and I, more preferably F and Cl, and most preferably F.

The term "halo" means a halogen atom, and is preferably, F, Cl, Br and I, more preferably F and Cl, and most preferably F.

The term "3- to 10-membered heterocyclic ring" denotes a non-aromatic ring system having 3 to 10 ring atoms, in which at least one ring atoms is a heteroatom.

"An effective amount" refers to an amount of a compound of the invention that confers a therapeutic effect on the treated subject. The therapeutic effect may be objective (i.e. measurable by some test or marker) or subjective (i.e. subject gives an indication of or feels an effect).

As used herein, the terms "administration" or "administering" mean a route of administration for a compound disclosed herein. Exemplary routes of administration include, but are not limited to, oral, intraocular, intravenous, intraperitoneal, intraarterial, and intramuscular. The preferred route of administration can vary depending on various factors, e.g. the components of the pharmaceutical composition comprising a compound disclosed herein, site of the potential or actual disease and severity of disease.

The terms "subject" and "patient" are used herein interchangeably. They refer to a human or another mammal (e.g., mouse, rat, rabbit, dog, cat, cattle, swine, sheep, horse or primate) that can be afflicted with or is susceptible to a disease or disorder but may or may not have the disease or disorder. It is preferred that the subject is human.

Compounds of the invention may be disclosed by the name or chemical structure. If a discrepancy exists between the name of a compound and its associated chemical structure, then the chemical structure prevails.

The invention will now be further illustrated by the following non-limiting examples. The specific examples below are to be construed as merely illustrative, and not limitative of the remainder of the disclosure in any way whatsoever. Without further elaboration, it is believed that one skilled in the art can, based on the description herein, utilise the present invention to its fullest extent. All references and publications cited herein are hereby incorporated by reference in their entirety.

**PREPARATION OF COMPOUNDS OF THE INVENTION**

The compounds of the invention can be prepared according to the following General Synthetic Procedures scheme by methods well known and appreciated in the art. Suitable reaction conditions are well known in the art and appropriate substitutions of solvents and co-reagents are within the common general knowledge of the person skilled in the art. Likewise, it will be appreciated by those skilled in the art that synthetic intermediates may be isolated and/or purified by various well-known techniques as needed or desired, and that frequently, it will be possible to use various intermediates directly in subsequent synthetic steps with little or no purification. Furthermore, the skilled person will appreciate that in some circumstances, the orders in which moieties are introduced is not critical. The particular order of steps required to produce the compounds of formula (I) is dependent upon the particular compound being synthesized, the starting compound, and the relative liability of the substituted moieties as is well appreciated by those of ordinary

skill in the art. All substituents, unless otherwise indicated, are as previously defined, and all reagents are well known and appreciated in the art.

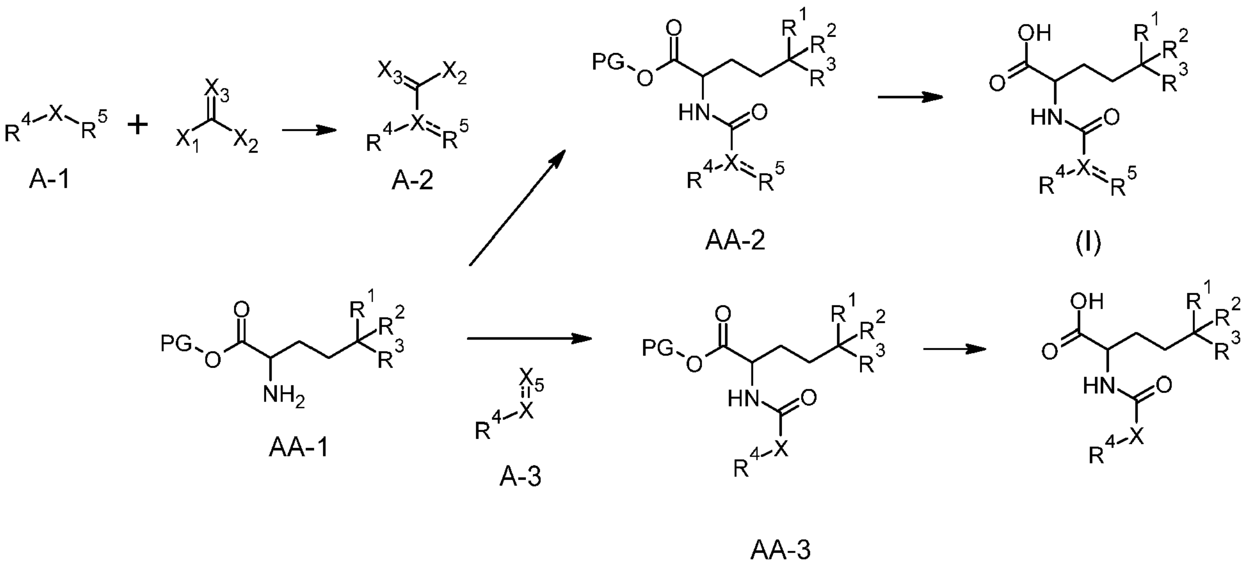

A-1 A-2 AA-2 (I) AA-1 A-3 AA-3

**General Synthetic Procedure, Scheme 1**

Suitable starting materials, either optically active as single enantiomers or as racemic mixtures and protected amino acids of general formula AA-1 are either commercially available or may be prepared by a variety of methods. For example, as illustrated in the General Synthetic Procedure, **Scheme 1,** the carboxylic acid functionality of appropriately substituted amino acids of general formula AA-1, can be used as the free acid, PG = H, or protected as a suitable derivative, for example as a methyl ester. Insertion of the substituent on the primary amine present in AA-1 can be done by a variety of methods, and for the purpose of exemplification, by a condensation step involving a suitable acyl reagent, for example but not limited to carbonyldiimidazole, phosgene or tri-phosgene, and the starting material AA-1 or the suitably substituted amine A-1 in a suitable solvent like, but not limited to dichloromethane. Those skilled in the art will realise that the urea or carbamate formation reaction can be performed in one single step or in successive steps in the same reactor or in separate successive steps.

**GENERAL SYNTHETIC PROCEDURES**

The compounds of general formula (I) may be prepared by a variety of procedures, some of which are described below. The products of each step can then be recovered by conventional methods including extraction, evaporation, precipitation, chromatography, filtration, trituration, crystallisation and the like.

Compounds of general formula (I) contain one or more stereogenic centres. Those can be introduced from available single enantiomers, optically active, starting materials of the type AA-1. The integrity of the existing stereogenic centre can be confirmed by analytical techniques well known to those skilled in the art like for example chiral support high pressure chromatography. Alternatively, when racemic starting materials are used, it will be appreciated that if desired, single isomer products can be obtained as single enantiomers or as single diastereoisomers, by known techniques like preparative chiral support high pressure chromatography.

The skilled artisan will also appreciate that not all of the substituents in the compounds of formula (I) will tolerate certain reaction conditions employed to synthesise the compounds. These moieties may be introduced at a convenient point in the synthesis, or may be protected and then deprotected as necessary or desired, as is well known in the art. The skilled artisan will appreciate that the protecting groups may be removed at any convenient point in the synthesis of the compounds of the present invention. Methods for introducing or removing protecting groups used in this invention are well known in the art; see, for example, Greene and Wuts, Protective Groups in Organic Synthesis, 4th Ed., John Wiley and Sons, New York (2006).

## EXAMPLES

### Abbreviations

approx: approximately; aq: aqueous; br: broad; *ca*.: *circa;* CDI: 1,1'-Carbonyldiimidazole; d: doublet; DCM: dichloromethane; DIC: *N,N'*-Di*i*sopropylcarbodiimide; dioxane: 1,4-dioxane; DIPEA: diisopropylethylamine; DMF: dimethylformamide; eq.: equivalent; $Et_3N$: triethylamine; EtOAc: ethyl acetate; EtOH: ethanol; Fmoc: fluorenylmethoxycarbonyl; Boc: *tert*butoxycarbonyl; h: hours; min: minutes: HATU: 2-(3*H*-[1,2,3]triazolo[4,5-b]pyridin-3-yl)-1,1,3,3-tetramethyl isouronium hexafluorophosphate(V); HPLC: high performance liquid chromatography; IPA, isopropanol; LC: liquid chromatography; m: multiplet; M: molar, molecular ion; MeCN: acetonitrile; MeOH: methanol; MS: mass spectrometry; NMR: nuclear magnetic resonance; PDA: photodiode array; q: quartet; rt: room temperature (ca. 20 °C); $R_T$: retention time; s: singlet, solid; SPPS: solid phase peptide synthesis. t: triplet; TBAF: tetrabutylammonium fluoride; TBME: *tert*-butyl methyl ether; TFA: trifluoroacetic acid; THF: tetrahydrofuran; UPLC: ultra-performance liquid chromatography; UV: ultraviolet.

Other abbreviations are intended to convey their generally accepted meaning.

### General Experimental Conditions

All starting materials and solvents were obtained either from commercial sources or prepared according to the literature citation. Reaction mixtures were magnetically stirred, and reactions performed at room temperature (ca. 20 °C) unless otherwise indicated.

Column chromatography was performed on an automated flash chromatography system, such as a CombiFlash Rf system, using pre-packed silica (40 μm) cartridges, unless otherwise indicated.

$^1$H-NMR spectra were recorded at 400 MHz on a Bruker Avance AV-I-400 or on a Bruker Avance AV-II-400 instrument. Chemical shift values are expressed in ppmvalues relative to tetramethylsilane unless noted otherwise. The following abbreviations or their combinations are used for multiplicity of NMR signals: br = broad, d = doublet, m = multiplet, q = quartet, quint = quintet, s = singlet and t = triplet.

### Example 1

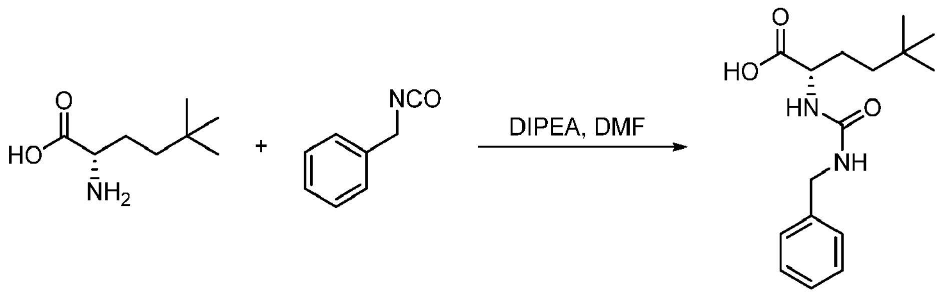


**Synthesis of (S)-2-(3-benzylureido)-5,5-dimethylhexanoic acid.**

Benzyl isocyanate (0.058 mL, 0.471 mmol) was added to a mixture of (S)-2-amino-5,5-dimethylhexanoic acid (50 mg, 0.314 mmol) and DIPEA (0.165 mL, 0.942 mmol) in N,N-Dimethylformamide (dry) (1 mL). The mixture was stirred at room temperature for 16 hours. The reaction mixture was purified by acidic prep (Method 2). The product containing fractions were combined and lyophillized. This resulted in (S)-2-(3-benzylureido)-5,5-dimethylhexanoic acid (57 mg, 0.195 mmol, 62 % yield) as white solid. LCMS (Method 1, 0.930 min; M+H = 293.1; calcd. 293.1). $^1$H-NMR (400 MHz, DMSO) δ 12.51 (s, 1H), 7.36 - 7.18 (m, 5H), 6.47 (t, *J* = 6.1 Hz, 1H), 6.21 (d, *J* = 8.2 Hz, 1H), 4.20 (d, *J* = 6.0 Hz, 2H), 4.14 - 4.04 (m, 1H), 1.70 - 1.58 (m, 1H), 1.58 - 1.45 (m, 1H), 1.22 - 1.13 (m, 2H), 0.85 (s, 9H).

Example 2

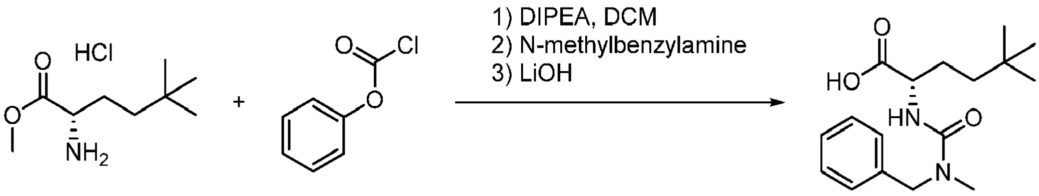


**Synthesis of (S)-2-(3-benzyl-3-methylureido)-5,5-dimethylhexanoic acid**

Phenyl chloroformate (0.073 ml, 0.577 mmol) was added to a mixture of methyl (S)-2-amino-5,5-dimethylhexanoate hydrochloride (100 mg, 0.477 mmol) and DIPEA (0.302 ml, 1.732 mmol) in Acetonitrile (anhydrous) (1 ml). The mixture was stirred for 4 h. N-Methylbenzylamine (0.112 ml, 0.866 mmol) was added. The mixture was stirred over 2 days. LiOH (75 mg, 3.13 mmol) and water (1 mL) were added. The mixture was stirred at 50 °C overnight. 1M HCl was added and the mixture was extracted with EtOAc. The solvent was evaporated and the product was purified by acidic prep (Method 2). The product containing fractions were combined and lyophillized to afford (S)-2-(3-benzyl-3-methylureido)-5,5-dimethylhexanoic acid (68.7 mg, 0.224 mmol, 46% yield). LCMS (Method 1, 0.985 min; M+H = 307.2; calcd. 307.2). $^1$H-NMR (400 MHz, DMSO) δ 12.36 (s, 1H), 7.35 - 7.29 (m, 2H), 7.27 - 7.23 (m, 1H), 7.23 - 7.19 (m, 2H), 6.41 (d, *J* = 7.9 Hz, 1H), 4.44 (dd, *J* = 22.2, 15.7 Hz, 2H), 4.00 (td, *J* = 8.7, 5.4 Hz, 1H), 2.77 (s, 3H), 1.75 -1.53 (m, 2H), 1.26 (td, *J* = 12.6, 4.8 Hz, 1H), 1.14 (td, *J* = 12.5, 5.0 Hz, 1H), 0.85 (s, 9H).

Example 3

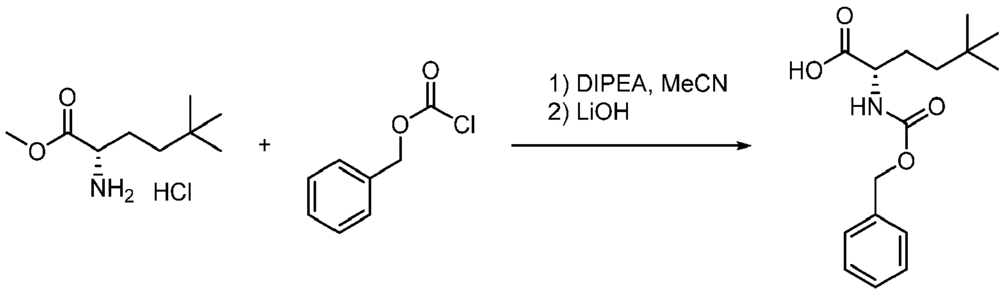


**Synthesis of (S)-2-(((benzyloxy)carbonyl)amino)-5,5-dimethylhexanoic acid**

Benzyl chloroformate (0.082 ml, 0.577 mmol) was added to a mixture of methyl (S)-2-amino-5,5-dimethylhexanoate hydrochloride (100 mg, 0.477 mmol) and DIPEA (0.302 ml, 1.732 mmol) in Acetonitrile (anhydrous) (1 ml). The mixture was stirred overnight at RT. 1 mL water and lithium hydroxide (75.0 mg, 3.13 mmol) were added and the mixture was stirred overnight at 40°C. The crude mixture was purified by acidic prep (Method 2). The product containing fractions were combined and lyophillized to afford (S)-2-(((benzyloxy)carbonyl)amino)-5,5-dimethylhexanoic acid (42.1 mg, 0.138 mmol, 28% yield). LCMS (Method 1, 1.025 min; M+H = 294.2; calcd. 294.2; M+H-$CO_2$ = 250.2; calcd. =250.2). $^1$H-NMR (400 MHz, DMSO) δ 12.57 (s, 1H), 7.56 (d, *J* = 8.1 Hz, 1H), 7.39 - 7.28 (m, 5H), 5.03 (s, 2H), 3.87 (td, *J* = 8.7, 4.6 Hz, 1H), 1.72 - 1.61 (m, 1H), 1.61 - 1.48 (m, 1H), 1.31 - 1.11 (m, 2H), 0.84 (s, 9H).

**Example 4**

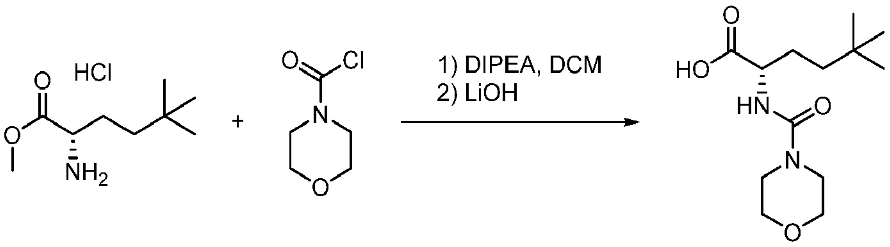


**Synthesis of (S)-5,5-dimethyl-2-(morpholine-4-carboxamido)hexanoic acid**

Morpholine-4-carbonyl chloride (178 mg, 1.19 mmol) was added to a solution of methyl (S)-2-amino-5,5-dimethylhexanoate hydrochloride (100 mg, 0.477 mmol) and DIPEA (0.291 mL, 1.67 mmol) in dichloromethane (1 mL). The solvent was removed. The product was taken up in 2 mL acetonitrile:water (1:1). Lithium hydroxide (57.1 mg, 2.38 mmol) was added and the mixture was stirred overnight at 40°C. The crude mixture was purified by acidic prep (Method 2). The product containing fractions were combined and lyophillized to afford (S)-5,5-dimethyl-2-(morpholine-4-carboxamido)hexanoic acid (97.3 mg, 0.357 mmol, 74% yield). LCMS (Method 1, 0.742 min; M+H = 273.2; calcd. 273.2). $^1$H-NMR (400 MHz, DMSO) δ 7.57-6.55 (br, 1H), 6.39 (d, *J* = 6.8 Hz, 1H), 3.85 - 3.74 (m, 1H), 3.54 (t, *J* = 4.9 Hz, 4H), 3.26 - 3.16 (m, 5H), 1.71 - 1.57 (m, 1H), 1.57 - 1.46 (m, 1H), 1.23 - 1.07 (m, 2H), 0.82 (s, 9H).

**Example 5**

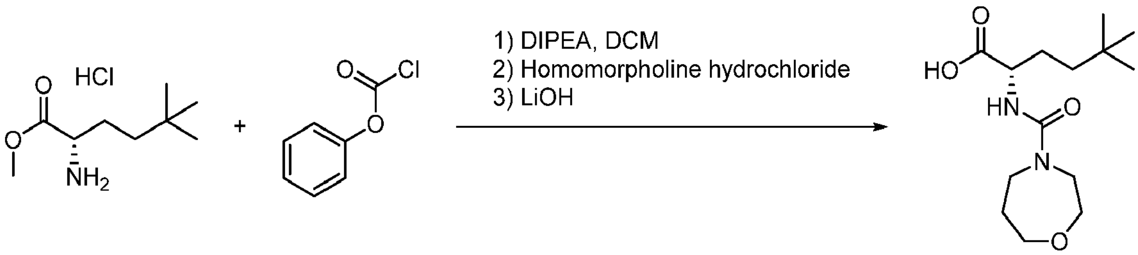


**Synthesis of (S)-5,5-dimethyl-2-(1,4-oxazepane-4-carboxamido)hexanoic acid**

Phenyl chloroformate (0.060 mL, 0.477 mmol) was added to a solution of methyl (S)-2-amino-5,5-dimethylhexanoate hydrochloride (100 mg, 0.477 mmol) and DIPEA (0.333 mL, 1.907 mmol) in dichloromethane (1 mL). The mixture was stirred for 1 h. Homomorpholine hydrochloride (131 mg, 0.954 mmol) was added and the mixture was stirred overnight. The solvent was removed The product was taken up in 2 mL acetonitrile:water (1:1). Lithium hydroxide (57.1 mg, 2.38 mmol) was added and the mixture was stirred overnight at 40°C. The crude mixture was purified by acidic prep (Method 2). The product containing fractions were combined and lyophillized to afford (S)-5,5-dimethyl-2-(1,4-oxazepane-4-carbxamido)hexanoic acid (74.7 mg, 0.261 mmol, 54 % yield). LCMS (Method 1, 0.752 min; M+H = 287.2; calcd. 287.2). $^1$H-NMR (400 MHz, DMSO) δ 7.67- 6.50 (br, 1H), 6.17 (d, *J* = 6.8 Hz, 1H), 3.84 (q, *J* = 6.6 Hz, 1H), 3.67 - 3.51 (m, 4H), 3.43 (q, *J* = 4.7 Hz, 4H), 1.85 - 1.71 (m, 2H), 1.69 - 1.58 (m, 1H), 1.59 - 1.46 (m, 1H), 1.20 - 1.07 (m, 2H), 0.83 (s, 9H).

**Method information**

**Method 1:** UPLC_AN_BASE, Apparatus: Waters IClass; Bin. Pump: UPIBSM, SM: UPISMFTN with SO; UPCMA, PDA: UPPDATC, 210-320 nm, SQD: ACQ-SQD2 ESI; ELSD: gaspressure 40 psi, drift tube temp: 50°C; column: Waters XSelect CSH C18, 50×2.1mm, 2.5μm, Temp: 25°C, Flow: 0.6 mL/min, Gradient: t0 = 5% B, t2.0min = 98% B, t2.7min = 98% B, Posttime: 0.3 min, Eluent A: 10mM ammonium bicarbonate in water (pH=9.5), Eluent B: acetonitrile.

**Method 2:** Apparatus: Agilent Technologies G6130B Quadrupole; HPLC instrument type: Agilent Technologies 1290 preparative LC; Column: Waters XSelect CSH (C18, 100x30mm, 10μ); Flow: 55 ml/min; Column temp: RT; Eluent A: 0.1% formid acid in water; Eluent B: 100% acetonitrile; Detection: DAD (220-320 nm); Detection: MSD (ESI pos/neg) mass range: 100 - 1000; fraction collection based on MS and DAD.

## BIOLOGICAL DATA

### Neurotensin scintillation proximity assay

The exemplified compounds of the invention were tested in a Neurotensin (NTS) scintillation proximity assay (SPA). The $IC_{50}$ data is shown in Table 1 below. NTS, which is a 13 amino acid neuropeptide, is a sortilin ligand. The $IC_{50}$ is a measure of the amount of the compound required to inhibit the binding of NTS to sortilin by 50%. The skilled person will recognise that the lower the $IC_{50}$ value, the less of the compound needed to achieve the desired effect, and as a result, the chances of undesirable off-target effects are reduced.

Compound affinity was determined by measuring the displacement of [$^3$H]-neurotensin binding to *h*-Sortilin in SPA format. Total volume of 40 µl in 50 mM HEPES pH 7.4 assay buffer containing 100 mM NaCl, 2.0 mM CaCl2, 0.1% BSA and 0.1% Tween-20. Compound pre-incubation for 30 minutes at room temperature with 150 nM of 6his-Sortilin before 5 nM [3H]-Neurotensin and Ni chelate imaging beads (Perkin Elmer) were added, after 6 hours the plate was read on a ViewLux with 360 s exposure time. Dose-response evaluation of compounds was performed with 8 concentrations of drugs (covering 3 decades). $IC_{50}$ values were calculated by nonlinear regression using the sigmoid concentration-response (variable slope) using CDD Vault software. All values reported are average of at least 2 determinations.

The data in Table 1 below shows that the compounds disclosed herein are sortilin inhibitors.

**Table 1**

| Example | IUPAC | SORTILIN Binding Assay [3H]NT SPA: IC50 (nM) |
|---|---|---|
| 1 | (2S)-2-[(benzylcarbamoyl)amino]-5,5-dimethylhexanoic acid | 380 |
| 2 | (2S)-2-{[benzyl(methyl)carbamoyl]amino}-5,5-dimethylhexanoic acid | 430 |
| 3 | (2S)-2-{[(benzyloxy)carbonyl]amino}-5,5-dimethylhexanoic acid | 560 |
| 4 | (2S)-5,5-dimethyl-2-[(morpholine-4-carbonyl)amino] hexanoic acid | 260 |
| 5 | (2S)-5,5-dimethyl-2-[(1,4-oxazepane-4-carbonyl)amino] hexanoic acid | 370 |

## REFERENCES

Andersen, J. et al., Identification of the first small-molecule ligand of the neuronal receptor sortilin and structure determination of the receptor-ligand complex. Acta Crystallogr D Biol Crystallogr (2014), 70(Pt 2), pp.451-460;

Baker, M. et al., Mutations in progranulin cause tau-negative frontotemporal dementia linked to chromosome 17. Nature (2006), 442(7105), pp. 916-919;

Brouwers, N. et al., Genetic variability in progranulin contributes to risk for clinically diagnosed Alzheimer disease. Neurology, (2008), 71(9), pp. 656-664;

Buttenshon, H.N. et al., Increased serum levels of sortilin are associated with depression and correlated with BDNF and VEGF, Nature Translational Psychiatry (2015), 5(e677), pp. 1-7;

Carecchio, M., et al., Cerebrospinal fluid biomarkers in Progranulin mutations carriers. J Alzheimers Dis (2011), 27(4), pp. 781-790;

Carrasquillo, M. et al., Genome-wide screen identifies rs646776 near sortilin as a regulator of progranulin levels in human plasma. Am J Hum Genet (2010), 87(6), pp. 890-897;

Chen, Z. Y. et al., Sortilin controls intracellular sorting of brain-derived neurotrophic factor to the regulated secretory pathway. J Neurosci (2005), 25(26), pp. 6156-6166;

Cruts, M. et al., Loss of progranulin function in frontotemporal lobar degeneration. Trends Genet (2008), 24(4), pp. 186-194;

De Muynck, L. et al., The neurotrophic properties of progranulin depend on the granulin E domain but do not require sortilin binding. Neurobiol Aging (2013), 34(11), pp. 2541-2547;

Egashira, Y. et al., The growth factor progranulin attenuates neuronal injury induced by cerebral ischemia-reperfusion through the suppression of neutrophil recruitment. J Neuroinflammation (2013), 10, pp. 105;
Galimberti, D. et al., GRN variability contributes to sporadic frontotemporal lobar degeneration. J Alzheimers Dis (2010), 19(1), pp. 171-177;
Galimberti, D.et al., Progranulin as a therapeutic target for dementia. Expert Opin Ther Targets (2018), 22(7), pp. 579-585. doi:10.1080/14728222.2018.1487951
Gass, J. et al., Progranulin regulates neuronal outgrowth independent of sortilin. Mol Neurodegener (2012), 7, pp. 33;
Gass, J. et al., Progranulin: an emerging target for FTLD therapies. Brain Res (2012), 1462, pp. 118-128;
Gijselinck, I. et al., Granulin mutations associated with frontotemporal lobar degeneration and related disorders: an update. Hum Mutat (2008), 29(12), pp. 1373-1386;
Goettsch, C. et al., Sortilin and Its Multiple Roles in Cardiovascular and Metabolic Diseases. Atherosclerosis, Thrombosis and Vascular Biology (2017), 38(1), pp. 19-25
Jansen, P. et al., Roles for the pro-neurotrophin receptor sortilin in neuronal development, aging and brain injury. Nature Neuroscience (2007), 10(11), pp.1449-1457;
Hu, F. et al., Sortilin-mediated endocytosis determines levels of the frontotemporal dementia protein, progranulin. Neuron (2010), 68(4), pp. 654-667;
Huang, G. et al., Insulin responsiveness of glucose transporter 4 in 3T3-L1 cells depends on the presence of sortilin. Mol Biol Cell (2013), 24 (19), pp.3115-3122;
Kaddai, V. et al. Involvement of TNF-$\alpha$ in abnormal adipocyte and muscle sortilin expression in obese mice and humans. Diabetologia (2009) 52, pp. 932-940;
Kjolby, M. et al., Sort1, encoded by the cardiovascular risk locus 1p13.3, is a regulator of hepatic lipoprotein export. Cell Metab (2010), 12 (3), pp. 213-223;
Laird, A. S. et al., Progranulin is neurotrophic in vivo and protects against a mutant TDP-43 induced axonopathy. PLoS One (2010), 5 (10), e13368;
Lee, W. et al., Targeted manipulation of the sortilin-progranulin axis rescues progranulin haploinsufficiency. Hum Mol Genet (2014), 23 (6), pp. 1467-1478;
Martens, L. et al., Progranulin deficiency promotes neuroinflammation and neuron loss following toxin-induced injury. J Clin Invest (2012), 122(11), pp. 3955-3959;
Mazella, J. et al., The 100-kDa neurotensin receptor is gp95/sortilin, a non-G-protein-coupled receptor. J Biol Chem (1998), 273(41), pp. 26273-26276;
Miyakawa, S. et al, Anti-sortilin1 Antibody Up-Regulates Progranulin via Sortilin1 Down-Regulation. Front Neurosci (2020), 14, pp. 586107;
Møller et al. Sortilin as a Biomarker for Cardiovascular Disease Revisited. Frontiers in Cardiovascular Medicine (2021), 8, 652584;
Mortensen, M.B. et al., Targeting sortilin in immune cells reduces proinflammatory cytokines and atherosclerosis. J Clin Invest (2014), 124(12), pp. 5317-5322;
Nykjaer, A. et al., Sortilin is essential for proNGF-induced neuronal cell death. Nature (2014), 427(6977), pp. 843-848;
Nykjaer, A., & Willnow, T. E, Sortilin: a receptor to regulate neuronal viability and function. Trends Neurosci (2012), 35(4), pp. 261-270.
Oh, T.J. et al., Circulating sortilin level as a potential biomarker for coronary atherosclerosis and diabetes mellitus. Cardiovascular Diabetology (2017), 16(92);
Pan, X. et al., Sortilin and retromer mediate retrograde transport of Glut4 in 3T3-L1 adipocytes. Mol Biol Cell (2017), 28(12), pp.1667-1675;
Petersen, C. et al., Molecular identification of a novel candidate sorting receptor purified from human brain by receptor-associated protein affinity chromatography. J Biol Chem (1997), 272(6), pp. 3599-3605;
Pickford, F. et al., Progranulin is a chemoattractant for microglia and stimulates their endocytic activity. Am J Pathol (2011), 178(1), pp. 284-295;
Pottier, C., et al., Potential genetic modifiers of disease risk and age at onset in patients with frontotemporal lobar degeneration and GRN mutations: a genome-wide association study. Lancet Neurol (2018), 17(6), pp. 548-558;
Quistgaard, E. et al., Ligands bind to Sortilin in the tunnel of a ten-bladed beta-propeller domain. Nat Struct Mol Biol (2009), 16(1), pp. 96-98;
Santos, A. M. et al., Sortilin Participates in Light-dependent Photoreceptor Degeneration in Vivo. PLoS ONE (2012), 7(4), pp. e36243-e36243.16. Kuruvilla, R. et al., A neurotrophin signaling cascade coordinates sympathetic neuron development through differential control of TrkA trafficking and retrograde signalling. Cell (2004), 118(2), pp. 243-255;
Shi, J. & Kandror, K. V., Sortilin Is Essential and Sufficient for the Formation of Glut4 Storage Vesicles in 3T3-L1 Adipocytes. Developmental Cell (2005), 9, pp. 99-108;
Sheng, J. et al., Progranulin polymorphism rs5848 is associated with increased risk of Alzheimer's disease. Gene (2014), 542(2), pp. 141-145;Skeldal, S. et al., Mapping of the Interaction Site between Sortilin and the p75 Neurotrophin Receptor Reveals a Regulatory Role for the Sortilin Intracellular Domain in p75 Neurotrophin Receptor Shedding and Apoptosis. J Biol Chem (2012), 21(287), pp. 43798-43809;
Tauris, J., et al., Proneurotrophin-3 May Induce Sortilin-Dependent Death In Inner Ear Neurons. Eur J Neuroscience (2020), 33(4), pp.622-31;
Tang, W. et al., The growth factor progranulin binds to TNF receptors and is therapeutic against inflammatory arthritis in mice. Science (2011), 332(6028), pp. 478-484;
Tao, J. et al., Neuroprotective effects of progranulin in ischemic mice. Brain Res (2012), 1436, pp. 130-136;
Tenk, H.K., et al., ProBDNF induces neuronal apoptosis via activation of a receptor complex of p75NTR and sortilin. J Neuroscience (2005), 10(11), pp.1449-1457
Van Kampen, J. M., et al., Progranulin gene delivery protects dopaminergic neurons in a mouse model of Parkinson's disease. PLoS One (2014), 9(5), e97032;
Willnow, T. E. et al., VPS10P-domain receptors - regulators of neuronal viability and function. Nat Rev Neurosci (2008), 9(12), pp. 899-909;
Willnow, T.E., et al., Sortilins: new players in lipoprotein metabolism. Current Opinion in Lipidology (2011), 22(2), pp. 79-85.
Wuts, P.G.M. and Greene, T.W, Greene's Protective Groups in Organic Synthesis, 4th Edition, John Wiley and Sons, New York (2006);
Xu, S.H. et al., Regional and Cellular Mapping of Sortilin Immunoreactivity in Adult Human Brain, Frotiers in Neuroanatomy (2019), 13(31), pp. 1-27;
Yano, H., et al., Proneurotrophin-3 is a neuronal apoptotic ligand: evidence for retrograde-directed cell killing. J Neurosci (2009), 29(47), pp. 14790-14802;
Yin, F., et al., Exaggerated inflammation, impaired host defense, and neuropathology in progranulin-deficient mice. J Exp Med (2010), 207(1), pp. 117-128;
Zheng, Y., et al., C-terminus of progranulin interacts with the beta-propeller region of sortilin to regulate progranulin trafficking. PLoS One (2011), 6(6), e21023;
Zhou, X. et al., Prosaposin facilitates sortilin-independent lysosomal trafficking of progranulin. J Cell Biol (2015), 210(6), pp. 991-1002.

## Claims

**1.** A compound of Formula (I)

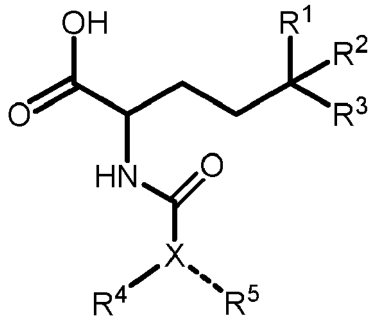

(I)

or a pharmaceutically acceptable salt, solvate, hydrate, tautomer, optical isomer, N-oxide, and/or prodrug thereof; wherein

X is selected from N and O;
$R^1$, $R^2$ and $R^3$ are each independently selected from the group consisting of halo, H, $(C_1-C_4)$alkyl, halo-$(C_1-C_4)$alkyl, $(C_2-C_4)$alkenyl, and halo-$(C_2-C_4)$alkenyl;
and
wherein $R^4$ is selected from the group consisting of $(C_3-C_{20})$aryl, halo-$(C_3-C_{20})$aryl, $(C_3-C_8)$heteroaryl, halo-$(C_3-C_{20})$heteroaryl, $(C_1-C_6)$-alkylene-$(C_3-C_{20})$-aryl, $(C_1-C_6)$-alkylene-$(C_3-C_{20})$-heteroaryl, $(C_1-C_6)$-alkylene-(3- to 10- membered-heterocyclic ring);

wherein the aryl group in $(C_1\text{-}C_6)$-alkylene-$(C_3\text{-}C_{20})$-aryl, the heteroaryl group in $(C_1\text{-}C_6)$-alkylene-$(C_3\text{-}C_{20})$-heteroaryl or the heterocyclic ring in $(C_1\text{-}C_6)$-alkylene-(3- to 8- membered heterocyclic ring) is optionally substituted with one or more substituents independently selected from halo, H, -OH, $(C_1\text{-}C_4)$alkyl, halo-$(C_1\text{-}C_4)$alkyl, $(C_1\text{-}C_4)$alkoxy and halo-$(C_1\text{-}C_4)$alkoxy;
wherein when X is O, $R^5$ is not present; and
when X is N, $R^5$ is selected from selected from the group consisting of halo, H, $(C_1\text{-}C_4)$alkyl, halo-$(C_1\text{-}C_4)$alkyl, $(C_2\text{-}C_4)$alkenyl, and halo-$(C_2\text{-}C_4)$alkenyl; or
wherein when X is N; X, $R^4$ and $R^5$ taken together form a 4- to 20-membered hererocyclic ring; wherein the heterocyclic ring is monocyclic, bicyclic or tricyclic and is optionally substituted with one or more substituents independently selected from halo, OH, cyano, carbonyl, $(C_1\text{-}C_4)$alkyl, halo-$(Ci\text{-}C_4)$alkyl, acetyl, $(C_1\text{-}C_4)$alkoxy, and halo-$(C_1\text{-}C_4)$alkoxy.

2. The compound according to claim 1, wherein $R^1$, $R^2$ and $R^3$ are each independently selected from the group consisting of halo, $(C_1\text{-}C_2)$alkyl and halo-$(C_1\text{-}C_2)$alkyl.

3. The compound according to claim 1 or claim 2, wherein $R^1$, $R^2$ and $R^3$ are each independently selected from F, $CH_3$ and $CF_3$.

4. The compound according to any preceding claim, wherein $R^4$ is selected from the group consisting of $(C_4\text{-}C_{10})$aryl, halo-$(C_4\text{-}C_{10})$aryl, $(C_3\text{-}C_8)$heteroaryl, halo-$(C_3\text{-}C_{20})$heteroaryl, $(C_1\text{-}C_4)$-alkylene-$(C_4\text{-}C_{10})$-aryl, $(C_1\text{-}C_4)$-alkylene-$(C_4\text{-}C_{10})$-heteroaryl, and $(C_1\text{-}C_4)$-alkylene-(3- to 10- membered-heterocyclic ring).

5. The compound according to any preceding claim, wherein $R^4$ is

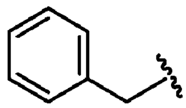

6. The compound according to any preceding claim, wherein X is N and $R^5$ is selected from the group consisting of H, $(C_1\text{-}C_3)$-alkyl and $(C_1\text{-}C_3)$haloalkyl.

7. The compound according to any of claims 1-3, wherein X is N; and X, $R^4$ and $R^5$ taken together form a 5- to 10-membered heterocyclic ring; wherein the heterocyclic ring is monocyclic or bicyclic and is optionally substituted with one or more substituents independently selected from halo, OH, carbonyl, $(C_1\text{-}C_3)$alkyl, halo-$(C_1\text{-}C_3)$alkyl, acetyl and $(C_1\text{-}C_3)$alkoxy, and halo-$(C_1\text{-}C_3)$alkoxy.

8. The compound according to claim 7, wherein X is N; and X, $R^4$ and $R^5$ taken together form:

(i) 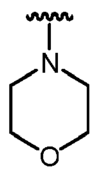 or (ii) 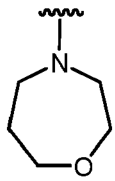 .

9. The compound according to any preceding claim, wherein the compound of Formula (I) is:

(2S)-2-[(benzylcarbamoyl)amino]-5,5-dimethylhexanoic acid;
(2S)-2-{[benzyl(methyl)carbamoyl]amino}-5,5-dimethylhexanoic acid;
(2S)-2-{[(benzyloxy)carbonyl]amino}-5,5-dimethylhexanoic acid;
(2S)-5,5-dimethyl-2-[(morpholine-4-carbonyl)amino]hexanoic acid; or (2S)-5,5-dimethyl-2-[(1,4-oxazepane-4-carbonyl)amino]hexanoic acid.

10. The compound according to any preceding claim, wherein the compound has a blood-to-brain $K_{puu}$ of more than 0.1.

11. A pharmaceutical composition comprising a compound according to any preceding claim and one or more pharmaceutically acceptable carriers, excipients, and/or diluents.

**12.** The compound according to any one of claims 1 to 10, or the pharmaceutical composition of claim 11, for use in therapy.

**13.** The compound according to any one of claims 1 to 10, or the pharmaceutical composition of claim 11, for use in the treatment or prevention of a neurodegenerative disorder, a psychiatric disorder, an inflammatory disorder, a cancer, pain, diabetes mellitus, brain tumours, diabetic retinopathy, glaucoma, chronic pain, uveitis, cardiovascular disease, kidney disease, psoriasis, hereditary eye conditions, hearing loss, diseases **characterized by** misfolded tau or a disease of the central nervous system.

**14.** The compound or pharmaceutical composition for use according to claim 13, wherein the neurodegenerative disorder is selected from frontotemporal dementia, Alzheimer's disease, Parkinson's disease, Huntington's disease, prion diseases such as Creutzfeldt-Jakob disease (CJD), acute brain injury, spinal cord injury and stroke;

wherein the psychiatric disorder is selected from bipolar disorder, major depression, post-traumatic stress disorder, and anxiety disorders.
wherein the inflammatory disorder is selected from inflammatory diseases and neuroinflammation;
wherein the cancer is selected from breast cancer, lung cancer, ovarian cancer, prostate cancer, thyroid cancer, pancreatic cancer, glioblastoma and colorectal cancer; and
wherein the hearing loss is selected from noise-induced hearing loss, ototoxicity induced hearing loss, age-induced hearing loss, idiopathic hearing loss, tinnitus and sudden hearing loss.

**15.** The compound or pharmaceutical composition for use according to claim 13 or claim 14, wherein the compound has a blood-to-brain $K_{puu}$ of more than 0.1; and wherein the compound or pharmaceutical composition is for use in the treatment or prevention of a disease of the central nervous system; preferably wherein the disease of the central nervous system is selected from:

a neurodegenerative disorder selected from frontotemporal dementia, Alzheimer's disease, Parkinson's disease, Huntington's disease, prion diseases such as Creutzfeldt-Jakob disease (CJD), acute brain injury, spinal cord injury and stroke;
a psychiatric disorder selected from bipolar disorder, major depression, post-traumatic stress disorder, and anxiety disorders;
hearing loss selected from noise-induced hearing loss, ototoxicity induced hearing loss, age-induced hearing loss, idiopathic hearing loss, tinnitus and sudden hearing loss;

brain tumours, retinopathies, glaucoma, neuroinflammation, chronic pain and diseases **characterized by** misfolded tau.

Europäisches Patentamt
European Patent Office
Office européen des brevets

# EUROPEAN SEARCH REPORT

Application Number
EP 22 15 9261

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE CAPLUS [Online] CHEMICAL ABSTRACTS SERVICE, COLUMBUS, OHIO, US; 1948, Huang et al.: "Chemical studies on amino acids and their derivatives. X. Syntheses of.alpha.-amino-.delta.-methylcaproic acid and .alpha.-aminocapric acid", XP002807084, Database accession no. 1948:23135 * abstract * & HUANG ET AL.: "Chemical studies on amino acids and their derivatives. X. Syntheses of.alpha.-amino-.delta.-methylcaproic acid and .alpha.-aminocapric acid", JOURNAL OF THE CHINESE CHEMICAL SOCIETY, vol. 1947, no. 15, 1947, - 1947, pages 46-54, Beijing<br>----- | 1,4,6,10 | INV.<br>A61P3/08<br>A61P9/00<br>A61P13/00<br>A61P17/06<br>A61P25/00<br>A61P27/02<br>A61P27/16<br>A61P35/00<br>A61P37/00<br>C07C271/22<br>C07C275/24<br>C07D295/205<br>A61K31/17<br>A61K31/27 |
| X | DATABASE CAPLUS [Online] CHEMICAL ABSTRACTS SERVICE, COLUMBUS, OHIO, US; 1978, Rozvaga, R. I.: "Chloro iodo derivatives of N-tolyl- and N-carb(alk)oxyalkyl(aryl)ureas", XP002807085, Database accession no. 1978:37390 * abstract * & ROZVAGA, R. I.: "Chloro iodo derivatives of N-tolyl- and N-carb(alk)oxyalkyl(aryl)ureas", UKRAINSKII KHIMICHESKII ZHURNAL (RUSSIAN EDITION), vol. 43, no. 8, 1977, - 1977, Kiev<br>-----<br>-/-- | 1,4,6,10 | TECHNICAL FIELDS SEARCHED (IPC)<br>A61P<br>C07C<br>C07D |

The present search report has been drawn up for all claims

| Place of search | Date of completion of the search | Examiner |
|---|---|---|
| Munich | 15 July 2022 | Panday, Narendra |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 22 15 9261

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE CAPLUS [Online] CHEMICAL ABSTRACTS SERVICE, COLUMBUS, OHIO, US; 2002, Dorsch, Dieter et al.: "Preparation of of ureido- and carbamoyloxy-substituted amides asinhibitors of factor Xa for the treatment of clotting disorders andtumors.", XP002807086, Database accession no. 2002:465965 * abstract * & WO 02/48099 A1 20 June 2002 (2002-06-20) ----- | 1,4,6,10 | |
| X | DATABASE CAPLUS [Online] CHEMICAL ABSTRACTS SERVICE, COLUMBUS, OHIO, US; 2004, Chen, S.: "The HPLC enantioresolution of phenyl isocyanated amino acids, peptides andamino alcohols on cyclodextrin bonded phases using the acetonitrile-basedmobile phase", XP002807087, Database accession no. 2004:686657 * abstract * & CHEN, S.: "The HPLC enantioresolution of phenyl isocyanated amino acids, peptides andamino alcohols on cyclodextrin bonded phases using the acetonitrile-basedmobile phase", CHROMATOGRAPHIA, vol. 59, no. 11, 2004, - 2004, pages 697-703, ----- | 1,4,6,10 | TECHNICAL FIELDS SEARCHED (IPC) |

-/--

The present search report has been drawn up for all claims

1

| Place of search | Date of completion of the search | Examiner |
|---|---|---|
| Munich | 15 July 2022 | Panday, Narendra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 15 9261

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2013/062947 A1 (ALLERGAN INC [US]; BEARD RICHARD L [US] ET AL.) 2 May 2013 (2013-05-02) * compound 7 on page 67 * | 1,4,6,10 | |
| X | US 2013/123496 A1 (BEARD RICHARD L [US] ET AL) 16 May 2013 (2013-05-16) * Page 32: last compound in right-hand col. Page 33: last compound in left-hand col. Page 34: 2nd compound in right-hand col. Page 35: 2nd compound in left-hand col. * | 1,4,6,10 | |
| X | CRASTO CURTIS F ET AL: "Synthesis and activity of analogues of the isoleucyl tRNA synthetase inhibitor SB-203207", BIOORGANIC, ELSEVIER, AMSTERDAM, NL, vol. 11, no. 13, 13 May 2003 (2003-05-13), pages 2687-2694, XP085050408, ISSN: 0968-0896, DOI: 10.1016/S0968-0896(03)00237-2 * Scheme 1: 6h, 6i * | 1,4-6,10 | TECHNICAL FIELDS SEARCHED (IPC) |
| X | DAWEI HUANG ET AL: "A novel series of-2-benzyloxycarbonylamino-8-(2-pyridyl)-disulfidyloctanoic acid derivatives as histone deacetylase inhibitors: Design, synthesis and molecular modeling study", EUROPEAN JOURNAL OF MEDICINAL CHEMISTRY, ELSEVIER, AMSTERDAM, NL, vol. 52, 6 March 2012 (2012-03-06), pages 111-122, XP028420641, ISSN: 0223-5234, DOI: 10.1016/J.EJMECH.2012.03.009 [retrieved on 2012-03-15] * Page 114, scheme 1, 2nd compound Z-L-Ab8 * | 1,4-6,10 | |

-/--

The present search report has been drawn up for all claims

1

| Place of search | Date of completion of the search | Examiner |
|---|---|---|
| Munich | 15 July 2022 | Panday, Narendra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 15 9261

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/070518 A1 (NAVITOR PHARM INC [US]) 27 April 2017 (2017-04-27)<br>* Final compounds of top scheme on page 78 and synthetic scheme on page 80 *<br>----- | 1-6,10 | |
| X | WO 2017/156074 A1 (BLADE THERAPEUTICS INC [US]) 14 September 2017 (2017-09-14)<br>* Compound 1f on page 226 *<br>----- | 1,4-6,10 | |
| X | DATABASE CA [Online]<br>CHEMICAL ABSTRACTS SERVICE, COLUMBUS, OHIO, US;<br>DOYLE, F. P. ET AL: "Derivatives of 6-aminopenicillanic acid. I. .alpha.-Amino-benzylpenicillin and some related compounds",<br>XP002807088,<br>retrieved from STN<br>Database accession no. 1962:462707<br>* abstract *<br>& DOYLE, F. P. ET AL: "Derivatives of 6-aminopenicillanic acid. I. .alpha.-Amino-benzylpenicillin and some related compounds",<br>JOURNAL OF THE CHEMICAL SOCIETY 1440-4 CODEN: JCSOA9; ISSN: 0368-1769,<br>1962,<br>DOI: 10.1039/JR9620001440<br>10.1039/JR9620001440<br>-----<br>-/-- | 1,4-6,10 | TECHNICAL FIELDS SEARCHED (IPC) |

The present search report has been drawn up for all claims

1

| Place of search | Date of completion of the search | Examiner |
|---|---|---|
| Munich | 15 July 2022 | Panday, Narendra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 15 9261

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MIYAZAWA TOSHIFUMI ET AL: "Porcine Pancreatic Lipase Catalyzed Enantioselective Hydrolysis of Esters of N -Protected Unusual Amino Acids", CHEMISTRY LETTERS, vol. 18, no. 12, 1 December 1989 (1989-12-01), pages 2219-2222, XP55942932, JP ISSN: 0366-7022, DOI: 10.1246/cl.1989.2219 Retrieved from the Internet: URL:http://dx.doi.org/10.1246/cl.1989.2219 > * Product of Scheme on page 2220 in view of table 1 on page 2221: Third to seventh entries for "R" * ----- | 1,4-6,10 | |
| X | DATABASE CA [Online] CHEMICAL ABSTRACTS SERVICE, COLUMBUS, OHIO, US; OJIMA, IWAO ET AL: "Design, synthesis and enzyme inhibitory activities of new trifluoromethyl-containing inhibitors for angiotensin converting enzyme", XP002807089, retrieved from STN Database accession no. 1994:630627 * abstract * & OJIMA, IWAO ET AL: "Design, synthesis and enzyme inhibitory activities of new trifluoromethyl-containing inhibitors for angiotensin converting enzyme", DRUG DESIGN AND DISCOVERY , 11(2), 91-113 CODEN: DDDIEV; ISSN: 1055-9612, 1994, ----- | 1-6,10 | TECHNICAL FIELDS SEARCHED (IPC) |

-/--

The present search report has been drawn up for all claims

1

| Place of search | Date of completion of the search | Examiner |
|---|---|---|
| Munich | 15 July 2022 | Panday, Narendra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 15 9261

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BALLINI R ET AL: "Base Assisted Substitution of @a-Amidoalkyl Sulfones by Nitromethane Anion. A New Entry to Functionalized @a-Amino Acids", TETRAHEDRON LETTERS, ELSEVIER, AMSTERDAM, NL, vol. 40, no. 23, 4 June 1999 (1999-06-04), pages 4449-4452, XP004164673, ISSN: 0040-4039, DOI: 10.1016/S0040-4039(99)00770-4 * Compound 85 on page 4450 * | 1,4-6,10 | |
| X | US 2004/180886 A1 (HICKEY EUGENE R [US] ET AL) 16 September 2004 (2004-09-16) * Page 13, top of right-hand column * | 1,2,7,8,10 | |
| X | Fukami Takehiro ET AL: "Synthesis and Structure?Activity Relationships of 2-Substituted d-Tryptophan-Containing Peptidic Endothelin Receptor Antagonists: Importance of the C-2 Substituent of the d-Tryptophan Residue for Endothelin A and B Receptor Subtype Selectivity", Journal of medicinal chemistry, 7 June 1996 (1996-06-07), pages 2313-2330, XP055853589, Washington, DC DOI: 10.1021/jm9600914 Retrieved from the Internet: URL:https://pubs.acs.org/doi/pdf/10.1021/jm9600914 [retrieved on 2021-10-21] * Compound 73c, 73e (norleucin, norvalin-based compounds) * | 1,7,10 | TECHNICAL FIELDS SEARCHED (IPC) |

-/--

The present search report has been drawn up for all claims

1

| Place of search | Date of completion of the search | Examiner |
|---|---|---|
| Munich | 15 July 2022 | Panday, Narendra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 15 9261

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | STACHEL SHAWN J ET AL: "Identification of potent inhibitors of the sortilin-progranulin interaction", BIOORGANIC & MEDICINAL CHEMISTRY LETTERS, ELSEVIER, AMSTERDAM, NL, vol. 30, no. 17, 15 July 2020 (2020-07-15), XP086232499, ISSN: 0960-894X, DOI: 10.1016/J.BMCL.2020.127403 [retrieved on 2020-07-15] * the whole document * ----- | 1-15 | |
| A | TENNA JUUL SCHRØDER ET AL: "The identification of AF38469: An orally bioavailable inhibitor of the VPS10P family sorting receptor Sortilin", BIOORGANIC & MEDICINAL CHEMISTRY LETTERS, vol. 24, no. 1, 27 November 2013 (2013-11-27), pages 177-180, XP055379658, AMSTERDAM, NL ISSN: 0960-894X, DOI: 10.1016/j.bmcl.2013.11.046 * the whole document * ----- | 1-15 | TECHNICAL FIELDS SEARCHED (IPC) |

The present search report has been drawn up for all claims

1

| Place of search | Date of completion of the search | Examiner |
|---|---|---|
| Munich | 15 July 2022 | Panday, Narendra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................
& : member of the same patent family, corresponding document

EPO FORM 1503 03.82 (P04C01)

# ANNEX TO THE EUROPEAN SEARCH REPORT ON EUROPEAN PATENT APPLICATION NO. EP 22 15 9261

This annex lists the patent family members relating to the patent documents cited in the above-mentioned European search report.
The members are as contained in the European Patent Office EDP file on
The European Patent Office is in no way liable for these particulars which are merely given for the purpose of information.

15-07-2022

| Patent document cited in search report | Publication date | Patent family member(s) | Publication date |
|---|---|---|---|
| WO 2013062947 A1 | 02-05-2013 | AR 088535 A1 | 18-06-2014 |
| | | AR 119698 A2 | 05-01-2022 |
| | | AU 2012329098 A1 | 22-05-2014 |
| | | AU 2017251683 A1 | 09-11-2017 |
| | | AU 2019264555 A1 | 05-12-2019 |
| | | BR 112014010042 A2 | 13-06-2017 |
| | | BR 122019025505 B1 | 22-02-2022 |
| | | CA 2853648 A1 | 02-05-2013 |
| | | CL 2014001080 A1 | 14-11-2014 |
| | | CN 104114164 A | 22-10-2014 |
| | | CN 106518742 A | 22-03-2017 |
| | | CO 6960509 A2 | 30-05-2014 |
| | | CY 1121604 T1 | 29-05-2020 |
| | | DK 2770989 T3 | 17-12-2018 |
| | | EP 2770989 A1 | 03-09-2014 |
| | | EP 3078656 A1 | 12-10-2016 |
| | | ES 2708997 T3 | 12-04-2019 |
| | | ES 2820714 T3 | 22-04-2021 |
| | | HK 1199622 A1 | 10-07-2015 |
| | | HR P20181934 T1 | 25-01-2019 |
| | | HU E040145 T2 | 28-02-2019 |
| | | IL 256555 A | 28-02-2018 |
| | | IL 267659 A | 29-08-2019 |
| | | JP 5982001 B2 | 31-08-2016 |
| | | JP 6496689 B2 | 03-04-2019 |
| | | JP 6804580 B2 | 23-12-2020 |
| | | JP 2014534216 A | 18-12-2014 |
| | | JP 2016216497 A | 22-12-2016 |
| | | JP 2019089849 A | 13-06-2019 |
| | | KR 20140111646 A | 19-09-2014 |
| | | LT 2770989 T | 10-12-2018 |
| | | MX 357737 B | 23-07-2018 |
| | | MY 172013 A | 12-11-2019 |
| | | NZ 624635 A | 27-01-2017 |
| | | PH 12019500171 A1 | 15-06-2020 |
| | | PL 2770989 T3 | 31-01-2019 |
| | | PT 2770989 T | 04-12-2018 |
| | | RU 2014120013 A | 10-12-2015 |
| | | RU 2017129434 A | 04-02-2019 |
| | | SG 10201903266Y A | 30-05-2019 |
| | | SG 11201401818R A | 29-05-2014 |
| | | SI 2770989 T1 | 31-12-2018 |
| | | TW 201331168 A | 01-08-2013 |
| | | UA 115043 C2 | 11-09-2017 |
| | | US 2013109866 A1 | 02-05-2013 |
| | | US 2014094614 A1 | 03-04-2014 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 22 15 9261

This annex lists the patent family members relating to the patent documents cited in the above-mentioned European search report. The members are as contained in the European Patent Office EDP file on
The European Patent Office is in no way liable for these particulars which are merely given for the purpose of information.

15-07-2022

| Patent document cited in search report | Publication date | Patent family member(s) | Publication date |
|---|---|---|---|
| | | US 2015148395 A1 | 28-05-2015 |
| | | US 2016235711 A1 | 18-08-2016 |
| | | US 2017119737 A1 | 04-05-2017 |
| | | US 2018228772 A1 | 16-08-2018 |
| | | US 2019343804 A1 | 14-11-2019 |
| | | US 2022202777 A1 | 30-06-2022 |
| | | WO 2013062947 A1 | 02-05-2013 |
| | | ZA 201403222 B | 29-07-2015 |
| US 2013123496 A1 | 16-05-2013 | AU 2012336013 A1 | 29-05-2014 |
| | | BR 112014011242 A2 | 25-05-2021 |
| | | CA 2855195 A1 | 16-05-2013 |
| | | CN 104169254 A | 26-11-2014 |
| | | EP 2776389 A1 | 17-09-2014 |
| | | EP 3088385 A1 | 02-11-2016 |
| | | HK 1199639 A1 | 10-07-2015 |
| | | JP 6181060 B2 | 16-08-2017 |
| | | JP 2015502924 A | 29-01-2015 |
| | | KR 20140117356 A | 07-10-2014 |
| | | RU 2014122750 A | 20-12-2015 |
| | | US 2013123496 A1 | 16-05-2013 |
| | | US 2013331570 A1 | 12-12-2013 |
| | | WO 2013070600 A1 | 16-05-2013 |
| WO 2017070518 A1 | 27-04-2017 | AU 2016342027 A1 | 10-05-2018 |
| | | AU 2021215177 A1 | 02-09-2021 |
| | | BR 112018007954 A2 | 30-10-2018 |
| | | CA 3001703 A1 | 27-04-2017 |
| | | CN 108289866 A | 17-07-2018 |
| | | CO 2018005315 A2 | 10-08-2018 |
| | | EP 3364958 A1 | 29-08-2018 |
| | | HK 1251486 A1 | 01-02-2019 |
| | | IL 258779 A | 28-06-2018 |
| | | JP 6800968 B2 | 16-12-2020 |
| | | JP 2018538248 A | 27-12-2018 |
| | | JP 2021050208 A | 01-04-2021 |
| | | KR 20180072704 A | 29-06-2018 |
| | | SG 11201803099S A | 30-05-2018 |
| | | US 2017114080 A1 | 27-04-2017 |
| | | US 2019048029 A1 | 14-02-2019 |
| | | US 2020079800 A1 | 12-03-2020 |
| | | US 2021047347 A1 | 18-02-2021 |
| | | WO 2017070518 A1 | 27-04-2017 |
| WO 2017156074 A1 | 14-09-2017 | AU 2017229591 A1 | 25-10-2018 |
| | | WO 2017156074 A1 | 14-09-2017 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 22 15 9261

This annex lists the patent family members relating to the patent documents cited in the above-mentioned European search report. The members are as contained in the European Patent Office EDP file on
The European Patent Office is in no way liable for these particulars which are merely given for the purpose of information.

15-07-2022

| Patent document cited in search report | Publication date | Patent family member(s) | Publication date |
|---|---|---|---|
| US 2004180886 A1 | 16-09-2004 | AR 043563 A1 | 03-08-2005 |
| | | AU 2004221860 A1 | 30-09-2004 |
| | | BR PI0408299 A | 07-03-2006 |
| | | CA 2518728 A1 | 30-09-2004 |
| | | CN 1761652 A | 19-04-2006 |
| | | EP 1606258 A1 | 21-12-2005 |
| | | JP 4455506 B2 | 21-04-2010 |
| | | JP 2006519768 A | 31-08-2006 |
| | | KR 20050109553 A | 21-11-2005 |
| | | MX PA05008240 A | 05-10-2005 |
| | | NZ 542905 A | 30-04-2009 |
| | | PE 20050312 A1 | 14-06-2005 |
| | | TW 200509919 A | 16-03-2005 |
| | | US 2004180886 A1 | 16-09-2004 |
| | | WO 2004083182 A1 | 30-09-2004 |
| | | ZA 200505418 B | 31-01-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20160331746 A
- WO 2021116290 A1
- EP 21152638
- EP 21194937

### Non-patent literature cited in the description


- **SILVERMAN, R. B.** The Organic Chemistry of Drug Design and Drug Action. Elsevier Academic Press, 2004, 498-549
- **GREENE ; WUTS.** Protective Groups in Organic Synthesis. John Wiley and Sons, 2006
- **ANDERSEN, J. et al.** Identification of the first small-molecule ligand of the neuronal receptor sortilin and structure determination of the receptor-ligand complex. *Acta Crystallogr D Biol Crystallogr,* 2014, vol. 70, 451-460
- **BAKER, M. et al.** Mutations in progranulin cause tau-negative frontotemporal dementia linked to chromosome 17. *Nature,* 2006, vol. 442 (7105), 916-919
- **BROUWERS, N. et al.** Genetic variability in progranulin contributes to risk for clinically diagnosed Alzheimer disease. *Neurology,* 2008, vol. 71 (9), 656-664
- **BUTTENSHON, H.N. et al.** Increased serum levels of sortilin are associated with depression and correlated with BDNF and VEGF. *Nature Translational Psychiatry,* 2015, vol. 5 (e677), 1-7
- **CARECCHIO, M. et al.** Cerebrospinal fluid biomarkers in Progranulin mutations carriers. *J Alzheimers Dis,* 2011, vol. 27 (4), 781-790
- **CARRASQUILLO, M. et al.** Genome-wide screen identifies rs646776 near sortilin as a regulator of progranulin levels in human plasma. *Am J Hum Genet,* 2010, vol. 87 (6), 890-897
- **CHEN, Z. Y. et al.** Sortilin controls intracellular sorting of brain-derived neurotrophic factor to the regulated secretory pathway. *J Neurosci,* 2005, vol. 25 (26), 6156-6166
- **CRUTS, M. et al.** Loss of progranulin function in frontotemporal lobar degeneration. *Trends Genet,* 2008, vol. 24 (4), 186-194
- **DE MUYNCK, L. et al.** The neurotrophic properties of progranulin depend on the granulin E domain but do not require sortilin binding. *Neurobiol Aging,* 2013, vol. 34 (11), 2541-2547
- **EGASHIRA, Y. et al.** The growth factor progranulin attenuates neuronal injury induced by cerebral ischemia-reperfusion through the suppression of neutrophil recruitment. *J Neuroinflammation,* 2013, vol. 10, 105
- **GALIMBERTI, D. et al.** GRN variability contributes to sporadic frontotemporal lobar degeneration. *J Alzheimers Dis,* 2010, vol. 19 (1), 171-177
- **GALIMBERTI, D. et al.** Progranulin as a therapeutic target for dementia. *Expert Opin Ther Targets,* 2018, vol. 22 (7), 579-585
- **GASS, J. et al.** Progranulin regulates neuronal outgrowth independent of sortilin. *Mol Neurodegener,* 2012, vol. 7, 33
- **GASS, J. E.** Progranulin: an emerging target for FTLD therapies. *Brain Res,* 2012, vol. 1462, 118-128
- **GIJSELINCK, I. et al.** Granulin mutations associated with frontotemporal lobar degeneration and related disorders: an update. *Hum Mutat,* 2008, vol. 29 (12), 1373-1386
- **GOETTSCH, C. et al.** Sortilin and Its Multiple Roles in Cardiovascular and Metabolic Diseases. *Atherosclerosis, Thrombosis and Vascular Biology,* 2017, vol. 38 (1), 19-25
- **JANSEN, P. et al.** Roles for the pro-neurotrophin receptor sortilin in neuronal development, aging and brain injury. *Nature Neuroscience,* 2007, vol. 10 (11), 1449-1457
- **HU, F. et al.** Sortilin-mediated endocytosis determines levels of the frontotemporal dementia protein, progranulin. *Neuron,* 2010, vol. 68 (4), 654-667
- **HUANG, G. et al.** Insulin responsiveness of glucose transporter 4 in 3T3-L1 cells depends on the presence of sortilin. *Mol Biol Cell,* 2013, vol. 24 (19), 3115-3122
- **KADDAI, V. et al.** Involvement of TNF-$\alpha$ in abnormal adipocyte and muscle sortilin expression in obese mice and humans. *Diabetologia,* 2009, vol. 52, 932-940

- **KJOLBY, M. et al.** Sort1, encoded by the cardiovascular risk locus 1p13.3, is a regulator of hepatic lipoprotein export. *Cell Metab,* 2010, vol. 12 (3), 213-223
- **LAIRD, A. S. et al.** Progranulin is neurotrophic in vivo and protects against a mutant TDP-43 induced axonopathy. *PLoS One,* 2010, vol. 5 (10), e13368
- **LEE, W. et al.** Targeted manipulation of the sortilin-progranulin axis rescues progranulin haploinsufficiency. *Hum Mol Genet,* 2014, vol. 23 (6), 1467-1478
- **MARTENS, L. et al.** Progranulin deficiency promotes neuroinflammation and neuron loss following toxin-induced injury. *J Clin Invest,* 2012, vol. 122 (11), 3955-3959
- **MAZELLA, J. et al.** The 100-kDa neurotensin receptor is gp95/sortilin, a non-G-protein-coupled receptor. *J Biol Chem,* 1998, vol. 273 (41), 26273-26276
- **MIYAKAWA, S. et al.** Anti-sortilin1 Antibody Up-Regulates Progranulin via Sortilin1 Down-Regulation. *Front Neurosci,* 2020, vol. 14, 586107
- **MØLLER et al.** Sortilin as a Biomarker for Cardiovascular Disease Revisited. *Frontiers in Cardiovascular Medicine,* 2021, vol. 8, 652584
- **MORTENSEN, M.B. et al.** Targeting sortilin in immune cells reduces proinflammatory cytokines and atherosclerosis. *J Clin Invest,* 2014, vol. 124 (12), 5317-5322
- **NYKJAER, A. et al.** Sortilin is essential for proNGF-induced neuronal cell death. *Nature,* 2014, vol. 427 (6977), 843-848
- **NYKJAER, A. ; WILLNOW, T. E.** Sortilin: a receptor to regulate neuronal viability and function. *Trends Neurosci,* 2012, vol. 35 (4), 261-270
- **OH, T.J. et al.** Circulating sortilin level as a potential biomarker for coronary atherosclerosis and diabetes mellitus. *Cardiovascular Diabetology,* 2017, vol. 16 (92
- **PAN, X. et al.** Sortilin and retromer mediate retrograde transport of Glut4 in 3T3-L1 adipocytes. *Mol Biol Cell,* 2017, vol. 28 (12), 1667-1675
- **PETERSEN, C. et al.** Molecular identification of a novel candidate sorting receptor purified from human brain by receptor-associated protein affinity chromatography. *J Biol Chem,* 1997, vol. 272 (6), 3599-3605
- **PICKFORD, F. et al.** Progranulin is a chemoattractant for microglia and stimulates their endocytic activity. *Am J Pathol,* 2011, vol. 178 (1), 284-295
- **POTTIER, C. et al.** Potential genetic modifiers of disease risk and age at onset in patients with frontotemporal lobar degeneration and GRN mutations: a genome-wide association study. *Lancet Neurol,* 2018, vol. 17 (6), 548-558
- **QUISTGAARD, E. et al.** Ligands bind to Sortilin in the tunnel of a ten-bladed beta-propeller domain. *Nat Struct Mol Biol,* 2009, vol. 16 (1), 96-98
- **SANTOS, A. M. et al.** Sortilin Participates in Light-dependent Photoreceptor Degeneration in Vivo. *PLoS ONE,* 2012, vol. 7 (4
- **KURUVILLA, R. et al.** A neurotrophin signaling cascade coordinates sympathetic neuron development through differential control of TrkA trafficking and retrograde signalling. *Cell,* 2004, vol. 118 (2), 243-255
- **SHI, J. ; KANDROR, K. V.** Sortilin Is Essential and Sufficient for the Formation of Glut4 Storage Vesicles in 3T3-L1 Adipocytes. *Developmental Cell,* 2005, vol. 9, 99-108
- **SHENG, J. et al.** Progranulin polymorphism rs5848 is associated with increased risk of Alzheimer's disease. *Gene,* 2014, vol. 542 (2), 141-145
- **SKELDAL, S. et al.** Mapping of the Interaction Site between Sortilin and the p75 Neurotrophin Receptor Reveals a Regulatory Role for the Sortilin Intracellular Domain in p75 Neurotrophin Receptor Shedding and Apoptosis. *J Biol Chem,* 2012, vol. 21 (287), 43798-43809
- **TAURIS, J. et al.** Proneurotrophin-3 May Induce Sortilin-Dependent Death In Inner Ear Neurons. *Eur J Neuroscience,* 2020, vol. 33 (4), 622-31
- **TANG, W. et al.** The growth factor progranulin binds to TNF receptors and is therapeutic against inflammatory arthritis in mice. *Science,* 2011, vol. 332 (6028), 478-484
- **TAO, J. et al.** Neuroprotective effects of progranulin in ischemic mice. *Brain Res,* 2012, vol. 1436, 130-136
- **TENK, H.K. et al.** ProBDNF induces neuronal apoptosis via activation of a receptor complex of p75NTR and sortilin. *J Neuroscience,* 2005, vol. 10 (11), 1449-1457
- **VAN KAMPEN, J. M. et al.** Progranulin gene delivery protects dopaminergic neurons in a mouse model of Parkinson's disease. *PLoS One,* 2014, vol. 9 (5), e97032
- **WILLNOW, T. E. et al.** VPS10P-domain receptors - regulators of neuronal viability and function. *Nat Rev Neurosci,* 2008, vol. 9 (12), 899-909
- **WILLNOW, T.E. et al.** Sortilins: new players in lipoprotein metabolism. *Current Opinion in Lipidology,* 2011, vol. 22 (2), 79-85
- **WUTS, P.G.M. ; GREENE, T.W.** Greene's Protective Groups in Organic Synthesis. John Wiley and Sons, 2006
- **XU, S.H. et al.** Regional and Cellular Mapping of Sortilin Immunoreactivity in Adult Human Brain. *Frotiers in Neuroanatomy,* 2019, vol. 13 (31), 1-27
- **YANO, H. et al.** Proneurotrophin-3 is a neuronal apoptotic ligand: evidence for retrograde-directed cell killing. *J Neurosci,* 2009, vol. 29 (47), 14790-14802

- **YIN, F. et al.** Exaggerated inflammation, impaired host defense, and neuropathology in progranulin-deficient mice. *J Exp Med,* 2010, vol. 207 (1), 117-128
- **ZHENG, Y. et al.** C-terminus of progranulin interacts with the beta-propeller region of sortilin to regulate progranulin trafficking. *PLoS One,* 2011, vol. 6 (6), e21023
- **ZHOU, X. et al.** Prosaposin facilitates sortilin-independent lysosomal trafficking of progranulin. *J Cell Biol,* 2015, vol. 210 (6), 991-1002